(19) Europäisches Patentamt
European Patent Office
Office européen des brevets

(11) EP 3 591 617 A1

(12) EUROPÄISCHE PATENTANMELDUNG

(43) Veröffentlichungstag:
08.01.2020 Patentblatt 2020/02

(51) Int Cl.:
G06T 11/00 (2006.01)    A61B 6/03 (2006.01)
A61B 6/00 (2006.01)

(21) Anmeldenummer: 18182251.1

(22) Anmeldetag: 06.07.2018

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(71) Anmelder: Siemens Healthcare GmbH
91052 Erlangen (DE)

(72) Erfinder:
• Birkhold, Annette
  90429 Nürnberg (DE)
• Kaethner, Christian
  91301 Forchheim (DE)
• Kowarschik, Markus
  90408 Nürnberg (DE)
• Rohkohl, Christopher
  AD700 Escaldes-Engordany (AD)

(54) **BESTIMMEN EINES DIFFERENZBILDDATENSATZES EINES UNTERSUCHUNGSVOLUMENS**

(57)    Die Erfindung betrifft eine Lösung zum Bestimmen eines dreidimensionalen Differenzbilddatensatzes eines Untersuchungsvolumens. Hierbei werden zweidimensionale Realbilddatensätze betreffend das Untersuchungsvolumen mittels einer Schnittstelle empfangen, wobei jeder der zweidimensionalen Realbilddatensätze eine zweidimensionale Röntgenprojektion des Untersuchungsvolumens bezüglich einer Projektionsrichtung umfasst. Weiterhin wird der erste Differenzbilddatensatz basierend auf den zweidimensionalen Realbilddatensätzen und basierend auf einer ersten trainierten Funktion mittels einer Recheneinheit bestimmt. Hierbei ist der erste Differenzbilddatensatz mindestens zweidimensional, insbesondere mindestens dreidimensional, insbesondere ist der erste Differenzbilddatensatz dreidimensional oder vierdimensional.

Durch das Bestimmen des ersten Differenzbilddatensatzes basierend auf den zweidimensionalen Realbilddatensätzen und basierend auf einer trainierten Funktion kann auf Maskenaufnahmen des Untersuchungsvolumens verzichtet werden kann, und somit die Röntgenbelastung des Untersuchungsvolumens verringert werden.

FIG 4

EP 3 591 617 A1

**Beschreibung**

In der digitalen Subtraktionsangiographie (kurz DSA) werden ein oder mehrere Gefäße in einem Untersuchungsvolumen durch Röntgenaufnahmen dargestellt, wobei zur Unterdrückung von weiteren Strukturen im Untersuchungsvolumen Aufnahmen des Gefäßes ohne Kontrastmittel (sog. Maskenaufnahmen) mit Aufnahmen des Gefäßes einschließlich eines Kontrastmittels, welches sich im Gefäß befindet, kombiniert werden. Das Kontrastmittel wird hierbei während der Untersuchung in das Gefäß eingebracht, um Parameter, insbesondere hydrodynamische Parameter eines Fluids zu bestimmen, wobei das Fluid im Gefäß fließt.

In der vierdimensionalen DSA wird mittels eines Bildrekonstruktionsverfahrens eine zeitaufgelöste Serie von dreidimensionalen DSA-Bilddaten bereitgestellt. Hierbei werden normierte zweidimensionale Röntgenprojektionen eines Untersuchungsvolumens zusammen mit einer Zeitinformation in ein Volumenelement rückprojiziert. Die zweidimensionalen Röntgenprojektionen entstammen hierbei üblicherweise einem rotierenden Aufnahmeprotokoll eines C-Arm-Röntgenbogens.

Dadurch, dass für eine digitale Subtraktionsangiographie sowohl Aufnahmen des Gefäßes ohne Kontrastmittel als auch Aufnahmen des Gefäßes einschließlich eines Kontrastmittels aufgenommen werden, wird das Untersuchungsvolumen einer hohen Röntgenbelastung ausgesetzt. Die Aufnahmen des Gefäßes ohne Kontrastmittel werden auch als Maskenaufnahmen bezeichnet.

Im Folgenden kann ein Bilddatensatz als Realbilddatensatz bezeichnet werden, wenn er die tatsächliche Verteilung von Werten und/oder Intensitäten (z.B. Hounsfield-Einheiten, Röntgenschwächungskoeffizienten) in einem Untersuchungsvolumen abbildet. Ein Bilddatensatz kann als Differenzbilddatensatz bezeichnet werden, wenn er eine Differenz einer tatsächlichen Verteilung von Werten und/oder Intensitäten in einem Untersuchungsvolumen abbildet. Ein Differenzbilddatensatz wird aber nicht notwendigerweise durch Subtraktion zweier Realbilddatensätze bestimmt. Ein Bilddatensatz kann als Subtraktionsbilddatensatz bezeichnet werden, wenn er durch Subtraktion zweier Bilddatensätze bestimmt wurde, insbesondere durch Subtraktion zweier Realbilddatensätze. Daher könnte insbesondere jeder Subtraktionsbilddatensatz als Differenzbilddatensatz aufgefasst werden, aber nicht jeder Differenzbilddatensatz kann als Subtraktionsbilddatensatz aufgefasst werden.

Es ist daher die Aufgabe der vorliegenden Erfindung, eine Lösung bereitzustellen, einen Differenzbilddatensatz ohne Maskenaufnahme bereitzustellen, und somit die Strahlenbelastung des Untersuchungsvolumens zu reduzieren.

Die Aufgabe wird gelöst durch ein Verfahren zum Bestimmen eines ersten Differenzbilddatensatzes, durch ein Verfahren zum Bestimmen eines zweidimensionalen Differenzbilddatensatzes, durch ein Bestimmungssystem, durch eine Röntgeneinheit, durch ein Verfahren zum Anpassen einer trainierten Funktion, durch ein Trainingssystem, durch ein Computerprogrammprodukt und durch ein computerlesbares Speichermedium. Bei den Verfahren kann es sich insbesondere um computerimplementierte Verfahren handeln.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Weiterhin wird die erfindungsgemäße Lösung der Aufgabe sowohl in Bezug auf Verfahren und Vorrichtungen zur Bestimmung von Differenzbilddatensätzen als auch in Bezug auf Verfahren und Vorrichtungen zum Anpassen von trainierten Funktionen beschrieben. Hierbei können Merkmale und alternative Ausführungsformen von Datenstrukturen und/oder Funktionen bei Verfahren und Vorrichtungen zur Bestimmung auf analoge Datenstrukturen und/oder Funktionen bei Verfahren und Vorrichtungen zum Anpassen übertragen werden. Analoge Datenstrukturen können hierbei insbesondere durch die Verwendung der Vorsilbe "Trainings" gekennzeichnet sein. Weiterhin können die in Verfahren und Vorrichtungen zur Bestimmung von Differenzbilddatensätzen verwendeten trainierten Funktionen insbesondere durch Verfahren und Vorrichtungen zum Anpassen von trainierten Funktionen angepasst worden und/oder bereitgestellt worden sein.

Die Erfindung basiert darauf, dass zweidimensionale Realbilddatensätze betreffend das Untersuchungsvolumen mittels einer Schnittstelle empfangen werden, wobei jeder der zweidimensionalen Realbilddatensätze eine zweidimensionale Röntgenprojektion des Untersuchungsvolumens bezüglich einer Projektionsrichtung umfasst. Weiterhin wird der erste Differenzbilddatensatz basierend auf den zweidimensionalen Realbilddatensätzen und basierend auf einer ersten trainierten Funktion mittels einer Recheneinheit bestimmt. Hierbei ist der erste Differenzbilddatensatz mindestens zweidimensional, insbesondere ist der erste Differenzbilddatensatz mindestens dreidimensional, insbesondere ist der erste Differenzbilddatensatz dreidimensional oder vierdimensional.

Hierbei kann das Untersuchungsvolumen wenigstens ein Gefäß umfassen, wobei das Gefäß ein Kontrastmittel enthalten kann, und wobei sich die räumliche Dichte bzw. die räumliche Verteilung des Kontrastmittels bei unterschiedlichen zweidimensionalen Röntgenprojektionen unterscheiden kann. Eine zweidimensionale Röntgenprojektion betreffend das Untersuchungsvolumen kann insbesondere eine zweidimensionale Röntgenprojektion des Untersuchungsvolumens sein. Insbesondere kann den zweidimensionalen Röntgenprojektionen ein Aufnahmezeitpunkt zugeordnet sein, dieser Aufnahmezeitpunkt entspricht dem Zeitpunkt der Aufnahme der zweidimensionalen Röntgenprojektion. Eine zweidimensionale Röntgenprojektion ist insbesondere räumlich zweidimensional.

Die zweidimensionalen Realbilddatensätze können insbesondere einen ersten zweidimensionalen Realbilddatensatz und einen zweiten zweidimensionalen Realbilddatensatz umfassen, wobei der erste zweidimensionale Realbilddatensatz eine erste Röntgenprojektion des Untersuchungsvolumens bezüglich einer ersten Projektionsrichtung umfasst, wobei der zweite zweidimensionale Realbilddatensatz eine zweite Röntgenprojektion des Untersuchungsvolumens bezüglich einer zweiten Projektionsrichtung umfasst, wobei sich die zweite Projektionsrichtung von der ersten Projektionsrichtung unterscheidet. Insbesondere können also die zweidimensionalen Realbilddatensätze eine erste Röntgenprojektion bezüglich einer ersten Projektionsrichtung und eine zweite Röntgenprojektion bezüglich einer zweiten Projektionsrichtung umfassen, wobei die erste Projektionsrichtung sich von der zweiten Projektionsrichtung unterscheidet. Die zweidimensionalen Realbilddatensätze können insbesondere Röntgenprojektionen bezüglich paarweise verschiedener Projektionsrichtungen umfassen. Es können insbesondere alle Projektionsrichtungen der Röntgenprojektionen der zweidimensionalen Realbilddatensätze eine gemeinsame Ebene aufspannen. Insbesondere bildet jeder der zweidimensionalen Realbilddatensätze die tatsächliche Verteilung von Werten und/oder Intensitäten (z.B. Hounsfield-Einheiten, Röntgenschwächungskoeffizienten) im Untersuchungsvolumen ab.

Eine trainierte Funktion bildet Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen oder der mehreren Parameter der trainierten Funktion kann insbesondere auf einem Paar aus Trainingseingabedaten und zugehörigen Trainingsausgabedaten basieren, wobei die trainierte Funktion zur Erzeugung von Trainingsabbildungsdaten auf die Trainingseingabedaten angewendet wird. Insbesondere können das Bestimmen und/oder das Anpassen auf einem Vergleich der Trainingsabbildungsdaten und der Trainingsausgabedaten basieren. Im Allgemeinen wird auch eine trainierbare Funktion, d.h. eine Funktion mit noch nicht angepassten einen oder mehreren Parametern, als trainierte Funktion bezeichnet.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk, wobei die Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion auch ein tiefes künstliches neuronales Netzwerk sein (ein englischer Fachbegriff ist "deep neural network" oder "deep artificial neural network"). Ein weiteres Beispiel für eine trainierte Funktion ist eine "Support Vector Machine", weiterhin sind auch insbesondere andere Algorithmen des maschinellen Lernens als trainierte Funktion einsetzbar.

Insbesondere wird der erste Differenzbilddatensatz basierend auf den zweidimensionalen Realbilddatensätzen und basierend auf einer ersten trainierten Funktion mittels einer Recheneinheit bestimmt, wenn die erste trainierte Funktion auf Eingabedaten angewendet wird und hierbei Ausgabedaten erzeugt, wobei die Eingabedaten auf den zweidimensionalen Realbilddatensätzen basieren, und wobei der erste Differenzbilddatensatz auf den Ausgabedatensätzen basiert. Insbesondere können die Eingabedaten identisch mit den zweidimensionalen Realbilddatensätzen sein. Insbesondere können hierbei die Ausgabedaten identisch mit dem ersten Differenzbilddatensatz sein.

Eine zweidimensionale Röntgenprojektion ist insbesondere räumlich zweidimensional. Ein erster Differenzbilddatensatz, der dreidimensional ist, kann insbesondere auch als "dreidimensionaler erster Differenzbilddatensatz" bezeichnet werden. Ein erster Differenzbilddatensatz, der vierdimensional ist, kann insbesondere auch als "vierdimensionaler erster Differenzbilddatensatz" bezeichnet werden. Ein dreidimensionaler erster Differenzbilddatensatz ist insbesondere räumlich dreidimensional. Ein vierdimensionaler erster Differenzbilddatensatz und/oder ein vierdimensionaler zweiter Differenzbilddatensatz ist insbesondere bezüglich dreier Raumrichtungen (ein anderes Wort ist "Raumdimension", "räumliche Dimension" oder "räumlicher Basisvektor") und bezüglich einer Zeitrichtung (ein anderes Wort ist "Zeitdimension", "zeitliche Dimension" oder "zeitlicher Basisvektor") ausgedehnt.

Insbesondere bildet der erste Differenzbilddatensatz eine Differenz einer tatsächlichen Verteilung von Werten und/oder Intensitäten im Untersuchungsvolumen ab. Der zweite Differenzbilddatensatz wird aber insbesondere nicht durch Subtraktion zweier Realbilddatensätze bestimmt. Insbesondere können im ersten Differenzbilddatensatz sonstige Strukturen im Untersuchungsbereich außerhalb des Gefäßes unterdrückt bzw. nicht enthalten bzw. nicht dargestellt sein. Insbesondere können auch die Ränder des Gefäßes im ersten Differenzbilddatensatz nicht enthalten und/oder unterdrückt sein. Insbesondere kann der erste Differenzbilddatensatz nur das Kontrastmittel bzw. die Kontrastmittelkonzentration darstellen, da (bis auf Artefakte) im Untersuchungsvolumen nur die Kontrastmittelkonzentration veränderlich ist.

Die Erfinder haben erkannt, dass auf Maskenaufnahmen und somit eine zusätzliche Röntgenbelastung des Untersuchungsvolumens verzichtet werden kann, wenn der erste Differenzbilddatensatzes basierend auf den zweidimensionalen Realbilddatensätzen und basierend auf einer trainierten Funktion bestimmt wird. Insbesondere ist also ein das Untersuchungsvolumen umfassender Patient einer kleineren Röntgenbelastung ausgesetzt. Ein anderes Wort für "Röntgenbelastung" ist "Strahlendosis".

Nach einem weiteren Aspekt der Erfindung ist der erste Differenzbilddatensatz dreidimensional, weiterhin umfasst das Verfahren das Rekonstruieren eines dreidimensionalen Realbilddatensatzes basierend auf den zweidimensionalen Realbilddatensätzen mittels der Recheneinheit. Weiterhin umfasst das Bestimmen des dreidimensionalen ersten Differenzbilddatensatzes ein Anwenden der ersten trainierten Funktion auf den dreidimensionalen Realbilddatensatz mittels der Recheneinheit.

Insbesondere basiert also das Bestimmen des ersten Differenzbilddatensatzes auf der ersten trainierten Funktion, indem das Bestimmen des ersten Differenzbilddatensatzes ein Anwenden der ersten trainierten Funktion auf den dreidimensionalen Realbilddatensatz umfasst. Insbesondere betrifft also dieser Aspekt der Erfindung ein Verfahren zum Bestimmen eines ersten Differenzbilddatensatzes eines Untersuchungsvolumens, wobei der erste Differenzbilddatensatz dreidimensional ist, das Verfahren umfassend:

- Empfangen von zweidimensionalen Realbilddatensätzen betreffend ein Untersuchungsvolumen mittels einer Schnittstelle, wobei jeder der zweidimensionalen Realbilddatensätze eine zweidimensionale Röntgenprojektion des Untersuchungsvolumens bezüglich einer Projektionsrichtung umfasst,
- Rekonstruieren eines dreidimensionalen Realbilddatensatzes basierend auf den zweidimensionalen Realbilddatensätzen mittels der Recheneinheit,
- Bestimmen des ersten Differenzbilddatensatzes basierend auf den zweidimensionalen Realbilddatensätzen mittels einer Recheneinheit, wobei das Bestimmen ein Anwenden der ersten trainierten Funktion auf den dreidimensionalen Realbilddatensatz umfasst.

Der dreidimensionale Realbilddatensatz bildet insbesondere eine tatsächliche Verteilung von Werten und/oder Intensitäten (z.B. Hounsfield-Einheiten, Röntgenschwächungskoeffizienten) im Untersuchungsvolumen ab. Die tatsächliche Verteilung von Werten und/oder Intensitäten ist hierbei insbesondere eine dreidimensionale tatsächliche Verteilung.

Die erste trainierte Funktion kann insbesondere auf den dreidimensionalen Realbilddatensatz angewendet werden, indem die Eingabedaten der ersten trainierten Funktion den dreidimensionalen Realbilddatensatz umfassen oder mit dem dreidimensionalen Realbilddatensatz identisch sind. Die erste trainierte Funktion kann insbesondere eine Funktion sein, welche dreidimensionale Bilddatensätze auf dreidimensionale Bilddatensätze abbildet.

Die Erfinder haben erkannt, dass der dreidimensionale Realbilddatensatz ein besonders geeigneter Eingabewert für die trainierte Funktion ist, denn er umfasst in geometrisch geordneter Weise und ohne unnötige Redundanzen alle in den Röntgenprojektionen der zweidimensionalen Realbilddatensätze enthaltenen Informationen über das Untersuchungsvolumen. Daher kann der erste Differenzbilddatensatz besonders effizient bestimmt werden.

Nach einem weiteren Aspekt der Erfindung umfasst das Verfahren weiterhin das Bestimmen eines dreidimensionalen Wahrscheinlichkeitsdatensatzes durch Anwendung der ersten trainierten Funktion auf den dreidimensionalen Realbilddatensatz mittels der Recheneinheit, und das Bestimmen des dreidimensionalen ersten Differenzbilddatensatzes umfasst ein pixelweises Multiplizieren des dreidimensionalen Wahrscheinlichkeitsdatensatzes mit dem dreidimensionalen Realbilddatensatz mittels der Recheneinheit.

Der dreidimensionale Wahrscheinlichkeitsdatensatz weist hierbei insbesondere einem oder mehreren Voxeln des dreidimensionalen Realbilddatensatzes einen Wahrscheinlichkeitswert zu.

Insbesondere kann der dreidimensionale Wahrscheinlichkeitswert allen Voxeln des dreidimensionalen Realbilddatensatzes einen Wahrscheinlichkeitswert zuweisen, in diesem Fall kann der dreidimensionale Wahrscheinlichkeitsdatensatz als dreidimensionaler Wahrscheinlichkeitsbilddatensatz aufgefasst werden. Ein Wahrscheinlichkeitswert ist insbesondere einen Zahl größer gleich 0 und kleiner gleich 1. Der einem Voxel zugeordnete Wahrscheinlichkeitswert kann insbesondere die Wahrscheinlichkeit betreffen, dass der Voxel im Abbild des im Untersuchungsvolumen befindlichen Gefäßes enthalten ist. Alternativ kann der einem Voxel zugeordnete Wahrscheinlichkeitswert insbesondere die Wahrscheinlichkeit betreffen, dass der Voxel nicht im Abbild des im Untersuchungsvolumen befindlichen Gefäßes enthalten ist.

Ein Wahrscheinlichkeitswert kann insbesondere auch binär sein, d.h. entweder den Wert 0 oder den Wert 1 aufweisen. In diesem Fall kann der Wahrscheinlichkeitsbilddatensatz auch als Segmentierung des Gefäßes im dreidimensionalen Realbilddatensatz aufgefasst werden.

Das Bestimmen des dreidimensionalen ersten Differenzbilddatensatzes kann insbesondere eine Multiplikation des dreidimensionalen Wahrscheinlichkeitsdatensatzes mit dem dreidimensionalen Realbilddatensatz umfassen, wenn der dreidimensionale erste Differenzbilddatensatz das Ergebnis der Multiplikation des dreidimensionalen Wahrscheinlichkeitsdatensatzes mit dem dreidimensionalen Realbilddatensatz ist und oder auf dem Ergebnis der Multiplikation des dreidimensionalen Wahrscheinlichkeitsdatensatzes mit dem dreidimensionalen Realbilddatensatz basiert. Die Multiplikation des dreidimensionalen Wahrscheinlichkeitsdatensatzes mit dem dreidimensionalen Realbilddatensatz kann insbesondere eine pixelweise Multiplikation sein.

Die Erfinder haben erkannt, dass mittels der Anwendung der ersten trainierten Funktion auf den dreidimensionalen Realbilddatensatz besonders einfach Wahrscheinlichkeitswerte dafür bestimmt werden können, dass bestimmte Voxel dem im Untersuchungsvolumen enthaltenem Gefäß entsprechen. Hierbei handelt es sich im weiteren Sinne um eine Bildverarbeitung, wobei trainierte Funktionen bekannter Weise gute Ergebnisse erzielen können. Durch die Multiplikation des dreidimensionalen Wahrscheinlichkeitsdatensatzes mit dem dreidimensionalen Realbilddatensatz kann dann effizient ein dreidimensionaler erster Differenzbilddatensatz erzeugt werden, da die Intensitätswerte von Bildbereichen mit niedrigen Wahrscheinlichkeitswerten durch die Multiplikation ausgeblendet werden, und diese Bildbereiche gerade den Bereichen des Untersuchungsvolumens entsprechen, die nicht dem im Untersuchungsvolumen enthaltenen Gefäß entsprechen.

Nach einem weiteren Aspekt der Erfindung umfasst das Verfahren weiterhin das Empfangen einer Transferfunktion mittels der Schnittstelle sowie das Modifizieren des dreidimensionalen Wahrscheinlichkeitsdatensatzes basierend auf der Transferfunktion mittels der Recheneinheit.

Eine Transferfunktion ist insbesondere eine Funktion, die Wahrscheinlichkeitswerte auf Wahrscheinlichkeitswerte abbildet. Insbesondere ist eine Transferfunktion daher eine Funktion, die das Interval [0; 1] auf das Interval [0; 1] abbildet. Insbesondere kann die Transferfunktion T eine monoton steigende Funktion sein, d.h. $T(x) \leq T(y)$ für $x < y$, insbesondere kann die Transferfunktion T auch eine streng monoton steigende Funktion sein, d.h. $T(x) < T(y)$ für $x < y$. Vorteilhafterweise ist die Transferfunktion eine stetige und/oder differenzierbare Funktion. Vorteilhafterweise gilt für die Transferfunktion T die Beziehungen $T(0) = 0$ und $T(1) = 1$.

Die Transferfunktion kann insbesondere durch einen Benutzer mittels einer Eingabeschnittstelle festgelegt werden. Alternativ kann die Transferfunktion auch aus einer Mehrzahl von verfügbaren Transferfunktionen ausgewählt werden, beispielsweise basierend auf der Art des dreidimensionalen Realbilddatensatzes, basierend auf Aufnahmeparametern für die zweidimensionalen Realbilddatensätze, basierend auf der Position des Untersuchungsvolumens im Körper des Patienten und/oder basierend auf dem im Untersuchungsvolumen enthaltenen Gefäß.

Das Modifizieren des dreidimensionalen Wahrscheinlichkeitsdatensatzes kann insbesondere eine Anwendung der Transferfunktion auf jeden Wahrscheinlichkeitswert des dreidimensionalen Wahrscheinlichkeitsdatensatzes umfassen. Insbesondere wird für jeden Wahrscheinlichkeitswert des dreidimensionalen Wahrscheinlichkeitsdatensatzes ein modifizierter Wahrscheinlichkeitswert bestimmt, indem die Transferfunktion auf den Wahrscheinlichkeitswert angewendet ist, und insbesondere umfasst der modifizierte dreidimensionale Wahrscheinlichkeitsdatensatz die modifizierten Wahrscheinlichkeitswerte.

Die Erfinder haben erkannt, dass durch die Anwendung einer geeigneten Transferfunktion Bildstrukturen bzw. die Intensität von Bildstrukturen, die dem Hintergrund entsprechen, verstärkt oder geschwächt werden können. Wird beispielsweise als Transferfunktion $T(x) = x^\gamma$ verwendet, so werden für $0 < \gamma < 1$ Bildstrukturen, die dem Hintergrund entsprechen, verstärkt, und für $\gamma > 1$ Bildstrukturen, die dem Hintergrund entsprechen, geschwächt.

Nach einem weiteren Aspekt der Erfindung ist der erste Differenzbilddatensatz dreidimensional, weiterhin umfasst das Verfahren das Bestimmen von zweidimensionalen Differenzbilddatensätzen durch Anwendung der ersten trainierten Funktion auf die zweidimensionalen Realbilddatensätze mittels der Recheneinheit, wobei das Bestimmen des dreidimensionalen ersten Differenzbilddatensatzes ein Rekonstruieren basierend auf den zweidimensionalen Differenzbilddatensätzen umfasst.

Insbesondere basiert also das Bestimmen des ersten Differenzbilddatensatzes auf den zweidimensionalen Realbilddatensätzen und der ersten trainierten Funktion, indem das Bestimmen des ersten Differenzbilddatensatzes ein Rekonstruieren des dreidimensionalen ersten Differenzbilddatensatzes basierend auf den zweidimensionalen Differenzbilddatensätzen umfasst, und die zweidimensionalen Differenzbilddatensätze ihrerseits auf den auf den zweidimensionalen Realbilddatensätzen und der ersten trainierten Funktion basierend. Insbesondere betrifft also dieser Aspekt der Erfindung ein Verfahren zum Bestimmen eines ersten Differenzbilddatensatzes eines Untersuchungsvolumens, wobei der ersten Differenzbilddatensatz dreidimensional ist, umfassend:

- Empfangen von zweidimensionalen Realbilddatensätzen betreffend ein Untersuchungsvolumen mittels einer Schnittstelle, wobei jeder der zweidimensionalen Realbilddatensätze eine zweidimensionale Röntgenprojektion des Untersuchungsvolumens bezüglich einer Projektionsrichtung umfasst,
- Bestimmen von zweidimensionalen Differenzbilddatensätzen durch Anwendung der ersten trainierten Funktion auf die zweidimensionalen Realbilddatensätze mittels der Recheneinheit,
- Bestimmen des ersten Differenzbilddatensatzes mittels einer Recheneinheit, wobei das Bestimmen des ersten Differenzbilddatensatzes ein Rekonstruieren basierend auf den zweidimensionalen Differenzbilddatensätzen umfasst.

Insbesondere werden zweidimensionale Differenzbilddatensätze durch Anwendung der zweiten trainierten Funktion auf die zweidimensionalen Realbilddatensätze bestimmt, wenn für das Bestimmen von jedem der zweidimensionalen Differenzbilddatensätze die erste trainierte Funktion auf genau einen der zweidimensionalen Realbilddatensätze angewendet wird. Insbesondere wird die erste trainierte Funktion auf einen zweidimensionalen Realbilddatensatz angewendet, wenn die Eingabedaten der ersten trainierten Funktion identisch mit den zweidimensionalen Realbilddatensätzen sind. Insbesondere können die Ausgabedaten einer solchen Anwendung dann identisch mit einem der zweidimensionalen Subtraktionsdatensätze sein. Insbesondere ist die erste trainierte Funktion eine Funktion, welche zweidimensionale Bilddatensätze auf zweidimensionale Bilddatensätze abbildet.

Die zweidimensionalen Differenzbilddatensätze bilden insbesondere eine Differenz einer tatsächlichen Verteilung von Werten und/oder Intensitäten im Untersuchungsvolumen ab. Die zweidimensionalen Differenzbilddatensätze werden aber insbesondere nicht durch Subtraktion zweier Realbilddatensätze bestimmt. Insbesondere können in den zweidimensionalen Differenzbilddatensätzen sonstige Strukturen im Untersuchungsbereich außerhalb des Gefäßes unterdrückt bzw. nicht enthalten bzw. nicht dargestellt sein. Insbesondere können auch die Ränder des Gefäßes in den zweidimensionalen Differenzbilddatensätzen nicht enthalten und/oder unterdrückt sein. Insbesondere kann die zweidimensionalen Differenzbilddatensätze nur das Kontrastmittel bzw. die Kontrastmittelkonzentration darstellen, da (bis auf Artefakte) im Untersuchungsvolumen nur die Kontrastmittelkonzentration veränderlich ist.

Die Erfinder haben erkannt, dass durch die Bestimmung von zweidimensionalen Differenzbilddatensätzen diese dann rückprojiziert werden können, und damit beispielsweise durch eine multiplikative Rückprojektion besonders genau höherdimensionale Differenzbilddatensätze bestimmt werden können.

Nach einem weiteren Aspekt der Erfindung ist der erste Differenzbilddatensatz vierdimensional. Bei diesem Aspekt erfolgt das Bestimmen des vierdimensionalen ersten Differenzbilddatensatzes durch Anwenden der ersten trainierten Funktion auf die zweidimensionalen Realbilddatensätze.

Ein vierdimensionaler Differenzbilddatensatz umfasst insbesondere eine Mehrzahl von dreidimensionalen Bilddatensätzen (insbesondere dreidimensionale Differenzbilddatensätze), wobei der vierdimensionale Differenzbilddatensatz insbesondere für jeden der zweidimensionalen Realbilddatensätze und/oder jeden der zweidimensionalen Differenzbilddatensätze einen dreidimensionalen Bilddatensatz umfasst, oder wobei der vierdimensionale Differenzbilddatensatz insbesondere für jeden zweidimensionalen Realbilddatensatz einer Untermenge der zweidimensionalen Realbilddatensätze und/oder für jeden zweidimensionalen Differenzbilddatensatz einer Untermenge der zweidimensionalen Differenzbilddatensätze einen dreidimensionalen Bilddatensatz umfasst. Insbesondere kann jeder der dreidimensionalen Bilddatensätze des vierdimensionalen Differenzbilddatensatzes eine Zeitinformation zugeordnet sein. Insbesondere wenn jeder der zweidimensionalen Realbilddatensätze und/oder jeder der zweidimensionalen Differenzbilddatensätze eine Zeitinformation umfasst, dann kann jede Zeitinformation eines dreidimensionalen Bilddatensatzes einer Zeitinformation der zweidimensionalen Realbilddatensätze und/oder jeder der zweidimensionalen Differenzbilddatensätze entsprechen. Insbesondere kann der vierdimensionale Differenzbilddatensatz bezüglich dreier räumlicher Dimensionen und einer zeitlichen Dimension ausgedehnt sein, insbesondere sind die im vierdimensionalen Differenzbilddatensatz enthaltenen dreidimensionalen Bilddatensätze bezüglich der drei räumlichen Dimensionen ausgedehnt. Der vierdimensionale Differenzbilddatensatz kann insbesondere eine zeitliche Entwicklung der Konzentration von Kontrastmittel im Gefäß im Untersuchungsvolumen darstellen.

Insbesondere ist in diesem Fall die erste trainierte Funktion eine Funktion, die eine Mehrzahl von zweidimensionalen Bilddatensätzen auf einen vierdimensionalen Bilddatensatz abbildet. Insbesondere kann die erste trainierte Funktion als weitere Eingabewerte noch Projektionsrichtungen und Zeitpunkte von Aufnahmen von zweidimensionalen Bilddatensätzen erhalten. Insbesondere umfassen in diesem Fall die Eingabedaten der ersten Funktion die zweidimensionalen Realbilddatensätze, und die Ausgabedaten der ersten Funktion umfassen den vierdimensionalen ersten Differenzbilddatensatz.

Die Erfinder haben erkannt, dass bei einer derartigen Bestimmung des vierdimensionalen ersten Differenzbilddatensatzes weitere Zwischenschritte in der Berechnung, wie beispielsweise eine Rekonstruktion, entfallen können. Alle Zwischenschritte sind bereits in der ersten trainierten Funktion enthalten. Dadurch kann das Bestimmen des vierdimensionalen ersten Differenzbilddatensatzes besonders effizient und schnell erfolgen.

Nach einem weiteren möglichen Aspekt der Erfindung umfasst das Verfahren weiterhin das Bestimmen eines vierdimensionalen zweiten Differenzbilddatensatzes basierend auf dem dreidimensionalen ersten Differenzbilddatensatz und den zweidimensionalen Realbilddatensätzen, oder basierend auf dem dreidimensionalen ersten Differenzbilddatensatz und den zweidimensionalen Differenzbilddatensätzen, mittels der Recheneinheit.

Der vierdimensionale zweite Differenzbilddatensatz kann dabei insbesondere alle vorteilhaften Aus- und Weiterbildungen des vierdimensionalen ersten Differenzbilddatensatzes aufweisen.

Die Erfinder haben erkannt, dass basierend auf dem dreidimensionalen Differenzbilddatensatz ein vierdimensionaler zweiter Differenzbilddatensatz erzeugt werden kann, ohne einen zusätzlichen Maskenlauf zur Aufnahme von Maskenaufnahmen durchführen zu müssen. Dadurch kann die Strahlenbelastung zur Erzeugung eines vierdimensionalen Differenzbilddatensatzes reduziert werden.

Nach einem weiteren Aspekt der Erfindung umfasst das Verfahren weiterhin das Bestimmen eines zweiten Differenzbilddatensatzes durch Anwendung einer zweiten trainierten Funktion auf Eingabedaten. Hierbei basieren die Eingabedaten auf den zweidimensionalen Realbilddatensätzen, den zweidimensionalen Differenzbilddatensätzen und/oder auf dem dreidimensionalen ersten Differenzbilddatensatz, weiterhin ist hierbei der zweite Differenzbilddatensatz vierdimensional.

Insbesondere können die Eingabedaten der zweiten trainierten Funktion auf den zweidimensionalen Realbilddatensätzen und dem dreidimensionalen ersten Differenzbilddatensatz basieren, oder mit den zweidimensionalen Realbilddatensätzen und dem dreidimensionalen ersten Differenzbilddatensatz identisch sein. Insbesondere können die Eingabedaten der zweiten trainierten Funktion auf den zweidimensionalen Differenzbilddatensätzen und dem dreidimensionalen ersten Differenzbilddatensatz basieren, oder mit den zweidimensionalen Differenzbilddatensätzen und dem dreidimensionalen ersten Differenzbilddatensatz identisch sein.

Die Erfinder haben erkannt, dass durch Anwendung der zweiten trainierten Funktion ein vierdimensionaler zweiter Differenzbilddatensatz erzeugt werden kann, ohne einen zusätzlichen Maskenlauf zur Aufnahme von Maskenaufnahmen durchführen zu müssen. Dadurch kann die Strahlenbelastung zur Erzeugung eines vierdimensionalen Differenzbilddatensatzes reduziert werden. Weiterhin kann durch die Verwendung einer zweiten trainierten Funktion der vierdimensionale Differenzbilddatensatz genauer und weniger fehleranfällig bestimmt werden, als beispielsweise durch die Verwendung einer Rückprojektion. Insbesondere können hierbei Ungenauigkeiten in der Bestimmung des dreidimensionalen Differenzbilddatensatzes ausgeglichen werden, weiterhin können alle Strukturen des dreidimensionalen Differenzbilddatensatzes bei der Bestimmung des vierdimensionalen Differenzbilddatensatzes berücksichtigt werden.

Nach einem weiteren möglichen Aspekt der Erfindung umfasst das Verfahren ein Bestimmen eines segmentierten dreidimensionalen ersten Differenzbilddatensatzes durch Segmentieren des dreidimensionalen ersten Differenzbilddatensatzes mittels der Recheneinheit. Insbesondere kann die Segmentierung durch Verwendung einer geeigneten Transferfunktion durchgeführt werden. Weiterhin umfasst dann das Bestimmen des vierdimensionalen zweiten Differenzbilddatensatzes eine Rückprojektion der zweidimensionalen Realbilddatensätze oder der zweidimensionalen Differenzbilddatensätze auf den segmentierten dreidimensionalen ersten Differenzbilddatensatz.

Beim Segmentieren des dreidimensionalen ersten Differenzbilddatensatzes wird der dreidimensionale erste Differenzbilddatensatz in wenigstens zwei Teile segmentiert bzw. aufgeteilt, wobei ein erster Teil wenigstens ein im Untersuchungsvolumen enthaltenes Gefäß und das Innere des Gefäßes umfasst, und ein zweiter Teil andere Bestandteile des Untersuchungsvolumen umfasst. Die Teile des segmentierten dreidimensionalen ersten Differenzbilddatensatzes können insbesondere disjunkt sein, insbesondere kann auch jeder Pixel des dreidimensionalen ersten Differenzbilddatensatzes durch die Segmentierung genau einem Teil zugewiesen werden. Insbesondere kann eine Segmentierung in genau zwei Teile erfolgen. Der erste Teil kann auch mehrere im Untersuchungsvolumen enthaltene Gefäße und das Innere der Gefäße umfassen.

Eine Rückprojektion ist ein Verfahren, das aus einem oder mehreren zweidimensionalen Projektionen eines dreidimensionalen Untersuchungsvolumens Daten betreffend das dreidimensionale Untersuchungsvolumen ermittelt. Bei den Daten betreffend das dreidimensionale Untersuchungsvolumen kann es sich insbesondere um Absorptionskoeffizienten oder Hounsfield-Einheiten handeln. Da eine zweidimensionale Projektion weniger Informationen enthält als das dreidimensionale Untersuchungsvolumen, können für eine Rückprojektion weitere Informationen verwendet werden, beispielsweise eine Segmentierung des Untersuchungsvolumens.

Die Erfinder haben erkannt, dass durch die Segmentierung und die Verwendung der Rückprojektion der vierdimensionale zweite Differenzbilddatensatz besonders effizient berechnet werden kann, insbesondere ist es hierbei nicht notwendig, für diesen Schritt Trainingsdaten zu gewinnen oder zu verwenden.

Nach einem weiteren Aspekt der Erfindung basieren die erste trainierte Funktion und/oder die zweite trainierte Funktion auf einem neuronalen Netzwerk. In anderen Worten basiert die erste trainierte Funktion auf einem ersten neuronalen Netzwerk, und/oder die zweite trainierte Funktion basiert auf einem zweiten neuronalen Netzwerk. Im Allgemeinen sind hierbei das erste neuronale Netzwerk und das zweite neuronale Netzwerk nicht identisch. Insbesondere kann eine trainierte Funktion auf einem neuronalen Netzwerk basieren, indem die trainierte Funktion identisch mit dem neuronalen Netzwerk ist oder die trainierte Funktion das neuronale Netzwerk umfasst.

Die Erfinder haben erkannt, dass neuronale Netzwerke besonders gut zur Bildverarbeitung, insbesondere für Segmentierungen, geeignet sind. Dadurch sind dann die erste trainierte Funktion und/oder die zweite trainierte Funktion besonders gut zum Bestimmen des dreidimensionalen Differenzbilddatensatzes geeignet.

Nach einem weiteren Aspekt der Erfindung umfasst das neuronale Netzwerk eine Faltungsschicht und/oder eine Entfaltungsschicht. Insbesondere umfasst das erste neuronale Netzwerk eine Faltungsschicht und/oder eine Entfaltungsschicht. Insbesondere umfasst das zweite neuronale Netzwerk eine Faltungsschicht und/oder eine Entfaltungsschicht. Insbesondere kann ein neuronales Netzwerk eine Sammelschicht umfassen (ein englischer Fachbegriff ist "pooling layer"). Insbesondere kann das erste neuronale Netzwerk und/oder das zweite neuronale Netzwerk eine Sammelschicht umfassen. Insbesondere kann ein neuronale Netzwerk ein faltendes neuronales Netzwerk sein (ein englischer Fachbegriff ist "convolutional neural network"). Insbesondere kann ein neuronales Netzwerk ein tiefes faltendes Netzwerk sein (ein englischer Fachbegriff ist "deep convolutional neural network")

Die Erfinder haben erkannt, dass durch die Verwendung von Faltungsschichten und/oder Entfaltungsschichten ein neuronales Netzwerk besonders effizient zur Bildverarbeitung eingesetzt werden, da trotz vieler Verbindungen zwischen Knotenschichten nur wenige Kantengewichte (nämlich die den Werten des Faltungskerns entsprechenden Kantengewichte) durch Training bestimmt werden müssen. Bei einer gleichen Zahl von Trainingsdaten kann damit auch die Genauigkeit des neuronalen Netzwerks verbessert werden.

Die Erfindung betrifft weiterhin ein Verfahren zum Bestimmen eines zweidimensionalen Differenzbilddatensatzes eines Untersuchungsvolumens, umfassend ein Empfangen eines zweidimensionalen Realbilddatensatzes betreffend das Untersuchungsvolumen mittels einer Schnittstelle, wobei der zweidimensionale Realbilddatensatz eine zweidimensionale Röntgenprojektion des Untersuchungsvolumens bezüglich einer Projektionsrichtung umfasst, weiterhin umfassend ein Bestimmen des zweidimensionalen Differenzbilddatensatzes durch Anwendung einer ersten trainierten Funktion auf den zweidimensionalen Realbilddatensatz mittels einer Recheneinheit.

Die Erfinder haben erkannt, dass mittels der ersten trainierten Funktion zweidimensionale Differenzbilddatensätze bestimmt werden können, ohne das Untersuchungsvolumen zusätzlicher Strahlungsbelastung durch eine Maskenaufnahme auszusetzen. Das Verfahren zum Bestimmen eines zweidimensionalen Differenzbilddatensatzes kann insbesondere auch Teil eines Verfahrens zum Bestimmen eines ersten Differenzbilddatensatzes sein, wobei der erste Differenzbilddatensatz mindestens dreidimensional ist, insbesondere dreidimensional oder vierdimensional.

Die Erfindung betrifft weiterhin ein Verfahren zum Anpassen einer ersten trainierten Funktion. Das Verfahren zum Anpassen der ersten trainierten Funktion basiert darauf, dass die erste trainierte Funktion mittels einer Schnittstelle empfangen wird. Weiterhin werden erste zweidimensionale Trainingsbilddatensätze und zweite zweidimensionale Trainingsbilddatensätze eines Untersuchungsvolumens mittels der Schnittstelle empfangen. Hierbei umfasst jeder der ersten zweidimensionalen Trainingsbilddatensätze eine erste zweidimensionale Röntgenprojektion des Untersuchungsvolumens bezüglich einer Projektionsrichtung, wobei das Untersuchungsvolumen während der Aufnahme der ersten zweidimensionalen Röntgenprojektion kein Röntgenkontrastmittel umfasst. Weiterhin umfasst hierbei jeder der zweiten zweidimensionalen Trainingsbilddatensätze eine zweite zweidimensionale Röntgenprojektion des Untersuchungsvolumens bezüglich einer Projektionsrichtung umfasst, wobei das Untersuchungsvolumen während der Aufnahme einer zweiten zweidimensionalen Röntgenprojektion Röntgenkontrastmittel umfasst. Das Verfahren zum Anpassen der ersten trainierten Funktion basiert weiterhin darauf, dass ein erster Trainingsdifferenzbilddatensatzes durch digitale Subtraktionsangiographie basierend auf den ersten und den zweiten zweidimensionalen Trainingsbilddatensätzen mittels einer Recheneinheit bestimmt wird. Das Verfahren zum Anpassen der ersten trainierten Funktion basiert weiterhin darauf, dass ein zweiter Trainingsdifferenzbilddatensatzes basierend auf den zweiten zweidimensionalen Trainingsbilddatensätzen und basierend auf der ersten trainierten Funktion mittels der Recheneinheit bestimmt wird. Das Verfahren zum Anpassen der ersten trainierten Funktion basiert weiterhin darauf, dass die trainierte Funktion basierend auf einem Vergleich des ersten dreidimensionalen Trainingsdifferenzbilddatensatzes und des zweiten dreidimensionalen Trainingsdifferenzbilddatensatzes mittels der Recheneinheit angepasst wird. Bei der ersten trainierten Funktion kann es sich insbesondere um die erste trainierte Funktion des Verfahrens zum Bestimmen eines Differenzbilddatensatzes handeln. Das Verfahren zum Anpassen der ersten trainierten Funktion kann weiterhin das Bereitstellen der ersten trainierten Funktion mittels der Schnittstelle umfassen.

Im Folgenden kann ein Bilddatensatz als Realbilddatensatz bezeichnet werden, wenn er die tatsächliche Verteilung von Werten und/oder Intensitäten (z.B. Hounsfield-Einheiten, Röntgenschwächungskoeffizienten) in einem Untersuchungsvolumen abbildet. Ein Bilddatensatz kann als Differenzbilddatensatz bezeichnet werden, wenn er eine Differenz einer tatsächlichen Verteilung von Werten und/oder Intensitäten in einem Untersuchungsvolumen abbildet. Ein Differenzbilddatensatz wird aber nicht notwendigerweise durch Subtraktion zweier Realbilddatensätze bestimmt. Ein Bilddatensatz kann als Subtraktionsbilddatensatz bezeichnet werden, wenn er durch Subtraktion zweier Bilddatensätze bestimmt wurde, insbesondere durch Subtraktion zweier Realbilddatensätze. Daher könnte insbesondere jeder Subtraktionsbilddatensatz als Differenzbilddatensatz aufgefasst werden, aber nicht jeder Differenzbilddatensatz kann als Subtraktionsbilddatensatz aufgefasst werden.

Insbesondere handelt es sich bei jedem der ersten zweidimensionalen Trainingsbilddatensätze um einen zweidimensionalen Realbilddatensatz, der insbesondere eine Differenz einer tatsächlichen Verteilung von Werten und/oder Intensitäten in einem Untersuchungsvolumen abbildet. Weiterhin handelt es sich insbesondere bei jedem der zweiten zweidimensionalen Trainingsbilddatensätze um einen zweidimensionalen Realbilddatensatz, der insbesondere eine Differenz einer tatsächlichen Verteilung von Werten und/oder Intensitäten in einem Untersuchungsvolumen abbildet.

Insbesondere handelt es sich bei dem ersten Trainingsdifferenzbilddatensatz um einen Differenzbilddatensatz, der insbesondere eine Differenz einer tatsächlichen Verteilung von Werten und/oder Intensitäten in einem Untersuchungsvolumen abbildet. Insbesondere handelt es sich beim ersten Trainingsdifferenzbilddatensatz um einen Subtraktionsbilddatensatz, der durch Subtraktion zweier Bilddatensätze bestimmt wurde, insbesondere durch Subtraktion zweier Realbilddatensätze. Insbesondere ist der erste Trainingsdifferenzbilddatensatz mindestens dreidimensional, insbesondere ist der erste Trainingsdifferenzbilddatensatz dreidimensional oder vierdimensional.

Weiterhin handelt es sich insbesondere bei dem zweiten Trainingsdifferenzbilddatensatz um einen Differenzbilddatensatz, der insbesondere eine Differenz einer tatsächlichen Verteilung von Werten und/oder Intensitäten in einem Untersuchungsvolumen abbildet, beim zweiten Trainingsdifferenzbilddatensatz handelt es sich aber nicht um einen Subtraktionsbilddatensatz. Insbesondere ist der zweite Trainingsdifferenzbilddatensatz mindestens dreidimensional, insbesondere ist der zweite Trainingsdifferenzbilddatensatz dreidimensional oder vierdimensional.

Insbesondere stimmen die Dimensionalität des ersten Trainingsdifferenzbilddatensatzes und die Dimensionalität des zweiten Trainingsdifferenzbilddatensatzes überein. Insbesondere stimmen die Ausdehnung des ersten Trainingsdifferenzbilddatensatzes und die Ausdehnung des zweiten Trainingsdifferenzbilddatensatzes bezüglich jeder Richtung bzw. jeder Dimension überein.

Die Erfinder haben erkannt, dass durch die Anwendung von digitaler Subtraktionsangiographie auf Bilddatensätze mit und ohne Kontrastmittel Differenzbilddatensätze bestimmt werden können, die mit den Ergebnissen basierend auf der ersten trainierten Funktion verglichen werden können. Dadurch können bereits vorhandene Daten bereits durchgeführter digitaler Subtraktionsangiographien zum Training der ersten trainierten Funktion verwendet werden. Dadurch ist es also nicht notwendig, separat Trainingsdaten aufzuzeichnen, insbesondere ist es also nicht notwendig, zur Generierung von Trainingsdaten zusätzliche Personen einer Strahlenbelastung auszusetzen, oder Patienten weiterer Strahlenbelastung auszusetzen.

Die Erfindung kann weiterhin ein Verfahren zum Anpassen einer zweiten trainierten Funktion betreffen, wobei das Verfahren die folgenden Schritte umfasst:

- Empfangen der zweiten trainierten Funktion mittels einer Schnittstelle,
- Empfangen eines dreidimensionalen Trainingsdifferenzbilddatensatzes eines Untersuchungsvolumens und von zweidimensionalen Trainingsbilddatensätzen des Untersuchungsvolumens mittels der Schnittstelle, wobei die zweidimensionalen Trainingsbilddatensätze zweidimensionalen Realbilddatensätzen oder zweidimensionalen Differenzbilddatensätzen entsprechen,
- Bestimmen eines ersten vierdimensionalen Trainingsdifferenzbilddatensatzes durch Rückprojektion basierend auf dem dreidimensionalen Trainingsdifferenzbilddatensatz und den zweidimensionalen Trainingsbilddatensätzen mittels einer Recheneinheit,
- Bestimmen eines zweiten vierdimensionalen Trainingsdifferenzbilddatensatzes durch Anwendung der zweiten trainierten Funktion auf den dreidimensionalen Trainingsdifferenzbilddatensatz und die zweidimensionalen Trainingsbilddatensätze mittels der Recheneinheit,
- Anpassen der zweiten trainierten Funktion basierend auf einem Vergleich des ersten vierdimensionalen Trainingsdifferenzbilddatensatzes und des zweiten vierdimensionalen Trainingsdifferenzbilddatensatzes mittels der Recheneinheit,
- optionales Bereitstellen der zweiten trainierten Funktion mittels der Schnittstelle.

Bei der zweiten trainierten Funktion kann es sich insbesondere um die zweite trainierte Funktion des Verfahrens zum Bestimmen eines Differenzbilddatensatzes handeln.

Die Erfinder haben erkannt dass durch dieses Verfahren die zweite Funktion besonders effizient trainiert werden kann, und eine solche trainierte Funktion besonders effizient zur Bestimmung von vierdimensionalen zweiten Differenzbilddatensätzen basierend auf dreidimensionalen ersten Differenzbilddatensätze eingesetzt werden kann.

Die Erfindung betrifft weiterhin ein Bestimmungssystem zum Bestimmen eines Differenzbilddatensatzes eines Untersuchungsvolumens, umfassend:

- Schnittstelle, ausgebildet zum Empfangen von zweidimensionalen Realbilddatensätzen betreffend ein Untersuchungsvolumen, wobei jeder der zweidimensionalen Realbilddatensätze eine zweidimensionale Röntgenprojektion des Untersuchungsvolumens bezüglich einer Projektionsrichtung umfasst,
- Recheneinheit, ausgebildet zum Bestimmen des Differenzbilddatensatzes basierend auf den zweidimensionalen Realbilddatensätzen und basierend auf einer trainierten Funktion, wobei der erste Differenzbilddatensatz mindestens zweidimensional, insbesondere mindestens dreidimensional ist.

Ein solches Bestimmungssystem kann insbesondere dazu ausgebildet sein die zuvor beschriebenen erfindungsgemäßen Verfahren zum Bestimmen eines Differenzbilddatensatzes und ihre Aspekte auszuführen. Das Bestimmungssystem ist dazu ausgebildet diese Verfahren und ihre Aspekte auszuführen, indem die Schnittstelle und die Recheneinheit ausgebildet sind, die entsprechenden Verfahrensschritte auszuführen.

Die Erfindung betrifft weiterhin eine Röntgeneinheit umfassend ein erfindungsgemäßes Bestimmungssystem. Die Röntgeneinheit kann insbesondere eine Röntgenquelle und/oder einen Röntgendetektor umfassen. Bei der Röntgeneinheit kann es sich insbesondere um ein C-Bogen-Röntgengerät handeln.

Die Erfindung kann weiterhin ein Bestimmungssystem zum Bestimmen eines zweidimensionalen Differenzbilddatensatzes eines Untersuchungsvolumens betreffen, umfassend:

- eine Schnittstelle, ausgebildet zum Empfangen eines zweidimensionalen Realbilddatensatzes betreffend das Untersuchungsvolumen, wobei der zweidimensionale Realbilddatensatz eine zweidimensionale Röntgenprojektion des Untersuchungsvolumens bezüglich einer Projektionsrichtung umfasst,
- eine Recheneinheit, ausgebildet zum Bestimmen des zweidimensionalen Differenzbilddatensatzes durch Anwendung einer ersten trainierten Funktion auf den zweidimensionalen Realbilddatensatz.

Die Erfindung betrifft weiterhin ein Trainingssystem zum Anpassen einer ersten trainierten Funktion, umfassend:

- Schnittstelle, ausgebildet zum Empfangen der ersten trainierten Funktion,
weiterhin ausgebildet zum Empfangen von ersten zweidimensionalen Trainingsbilddatensätzen und von zweiten zweidimensionalen Trainingsbilddatensätzen eines Untersuchungsvolumens, wobei jeder der ersten zweidimensionalen Trainingsbilddatensätze eine zweidimensionale Röntgenprojektion des Untersuchungsvolumens bezüglich einer Projektionsrichtung umfasst, wobei das Untersuchungsvolumen während der Aufnahme einer ersten zweidimensionalen Röntgenprojektion kein Röntgenkontrastmittel umfasst, wobei jeder der zweiten zweidimensionalen Trainingsbilddatensätze eine zweite zweidimensionale Röntgenprojektion des Untersuchungsvolumens bezüglich einer Projektionsrichtung umfasst, und wobei das Untersuchungsvolumen während der Aufnahme einer zweiten Röntgenprojektion Röntgenkontrastmittel umfasst,
- Recheneinheit, ausgebildet zum Bestimmen eines ersten Trainingsdifferenzbilddatensatzes durch digitale Subtraktionsangiographie basierend auf den ersten und den zweiten zweidimensionalen Trainingsbilddatensätzen,

weiterhin ausgebildet zum Bestimmen eines zweiten Trainingsdifferenzbilddatensatzes basierend auf den zweiten zweidimensionalen Trainingsbilddatensätzen und basierend auf der ersten trainierten Funktion,
weiterhin ausgebildet zum Anpassen der ersten trainierten Funktion basierend auf einem Vergleich des ersten Trainingsdifferenzbilddatensatzes und des zweiten Trainingsdifferenzbilddatensatzes.

Ein solches Trainingssystem kann insbesondere dazu ausgebildet sein, die zuvor beschriebenen erfindungsgemäßen Verfahren zum Anpassen einer trainierten Funktion und ihre Aspekte auszuführen. Das Trainingssystem ist dazu ausgebildet diese Verfahren und ihre Aspekte auszuführen, indem die Schnittstelle und die Recheneinheit ausgebildet sind, die entsprechenden Verfahrensschritte auszuführen.

Die Erfindung kann weiterhin ein Trainingssystem zum Anpassen einer zweiten trainierten Funktion betreffen, umfassend:

- Schnittstelle, ausgebildet zum Empfangen der zweiten trainierten Funktion,
weiterhin ausgebildet zum Empfangen eines dreidimensionalen Trainingsdifferenzbilddatensatzes eines Untersuchungsvolumens und von zweidimensionalen Trainingsbilddatensätzen des Untersuchungsvolumens, wobei die zweidimensionalen Trainingsbilddatensätze zweidimensionalen Realbilddatensätzen oder zweidimensionalen Differenzbilddatensätzen entsprechen,
optional weiterhin ausgebildet zum Bereitstellen der zweiten trainierten Funktion,
- Recheneinheit, ausgebildet zum Bestimmen eines ersten vierdimensionalen Trainingsdifferenzbilddatensatzes durch Rückprojektion basierend auf dem dreidimensionalen Trainingsdifferenzbilddatensatz und den zweidimensionalen Trainingsbilddatensätzen,

weiterhin ausgebildet zum Bestimmen eines zweiten vierdimensionalen Trainingsdifferenzbilddatensatzes durch Anwendung der zweiten trainierten Funktion auf den dreidimensionalen Trainingsdifferenzbilddatensatz und die zweidimensionalen Trainingsbilddatensätze,
weiterhin ausgebildet zum Anpassen der zweiten trainierten Funktion basierend auf einem Vergleich des ersten vierdimensionalen Trainingsdifferenzbilddatensatzes und des zweiten vierdimensionalen Trainingsdifferenzbilddatensatzes.

Die Erfindung betrifft auch Computerprogrammprodukte mit Computerprogrammen sowie computerlesbare Medien.

Insbesondere betrifft die Erfindung ein Computerprogrammprodukt mit einem Computerprogramm, welches direkt in einen Speicher eines Bestimmungssystems und/oder eines Trainingssystems ladbar ist, mit Programmabschnitten, um alle Schritte des Verfahrens zum Bestimmen eines ersten Differenzbilddatensatzes nach einem der Ansprüche 1 bis 9 und/oder um alle Schritte des Verfahrens zum Bestimmen eines zweidimensionalen Differenzbilddatensatzes nach dem Anspruch 10 und/oder um alle Schritte des Verfahrens zum Trainieren einer ersten trainierten Funktion nach dem Anspruch 11 auszuführen, wenn die Programmabschnitte von dem Bestimmungssystem und/oder dem Trainingssystem ausgeführt werden.

Insbesondere kann die Erfindung auch ein Computerprogrammprodukt mit einem Computerprogramm betreffen, welches direkt in einen Speicher eines Bestimmungssystems ladbar ist, mit Programmabschnitten, um alle Schritte des Verfahrens zum Bestimmen eines ersten Differenzbilddatensatzes nach einem der Ansprüche 1 bis 9 auszuführen, wenn die Programmabschnitte von dem Bestimmungssystem ausgeführt werden.

Insbesondere kann die Erfindung auch ein Computerprogrammprodukt mit einem Computerprogramm betreffen, welches direkt in einen Speicher eines Bestimmungssystems ladbar ist, mit Programmabschnitten, um alle Schritte des Verfahrens zum Bestimmen eines zweidimensionalen Differenzbilddatensatzes nach dem Anspruch 10 auszuführen, wenn die Programmabschnitte von dem Bestimmungssystem ausgeführt werden.

Insbesondere kann die Erfindung auch ein Computerprogrammprodukt mit einem Computerprogramm betreffen, welches direkt in einen Speicher eines Trainingssystems ladbar ist, mit Programmabschnitten, um alle Schritte des Verfahrens zum Trainieren einer ersten trainierten Funktion nach dem Anspruch 11 auszuführen, wenn die Programmabschnitte von dem Trainingssystem ausgeführt werden.

Insbesondere kann die Erfindung auch ein Computerprogrammprodukt mit einem Computerprogramm betreffen, welches direkt in einen Speicher eines Trainingssystems ladbar ist, mit Programmabschnitten, um alle Schritte des Verfahrens zum Trainieren einer zweiten trainierten Funktion auszuführen, wenn die Programmabschnitte von dem Trainingssystem ausgeführt werden.

Insbesondere betrifft die Erfindung ein computerlesbares Speichermedium, auf welchem von einem Bestimmungssystem und/oder einem Trainingssystem lesbare und ausführbare Programmabschnitte gespeichert sind, um alle Schritte des Verfahrens zum Bestimmen eines ersten Differenzbilddatensatzes nach einem der Ansprüche 1 bis 9 und/oder um alle Schritte des Verfahrens zum Bestimmen eines zweidimensionalen Differenzbilddatensatzes nach dem Anspruch 10 und/oder um alle Schritte des Verfahrens zum Trainieren einer ersten trainierten Funktion nach dem Anspruch 11 auszuführen, wenn die Programmabschnitte von dem Bestimmungssystem und/oder dem Trainingssystem ausgeführt werden.

Insbesondere kann die Erfindung auch ein computerlesbares Speichermedium betreffen, auf welchem von einem Bestimmungssystem lesbare und ausführbare Programmabschnitte gespeichert sind, um alle Schritte des Verfahrens zum Bestimmen eines ersten Differenzbilddatensatzes nach einem der Ansprüche 1 bis 9 auszuführen, wenn die Programmabschnitte von dem Bestimmungssystem ausgeführt werden.

Insbesondere kann die Erfindung auch ein computerlesbares Speichermedium betreffen, auf welchem von einem Bestimmungssystem lesbare und ausführbare Programmabschnitte gespeichert sind, um alle Schritte des Verfahrens zum Bestimmen eines zweidimensionalen Differenzbilddatensatzes nach dem Anspruch 10 auszuführen, wenn die Programmabschnitte von dem Bestimmungssystem ausgeführt werden.

Insbesondere kann die Erfindung auch ein computerlesbares Speichermedium betreffen, auf welchem von einem Trainingssystem lesbare und ausführbare Programmabschnitte gespeichert sind, um alle Schritte des Verfahrens zum Trainieren einer ersten trainierten Funktion nach dem Anspruch 11 auszuführen, wenn die Programmabschnitte von dem Trainingssystem ausgeführt werden.

Insbesondere kann die Erfindung auch ein computerlesbares Speichermedium betreffen, auf welchem von einem Trainingssystem lesbare und ausführbare Programmabschnitte gespeichert sind, um alle Schritte des Verfahrens zum Trainieren einer zweiten trainierten Funktion auszuführen, wenn die Programmabschnitte von dem Trainingssystem ausgeführt werden.

Eine weitgehend softwaremäßige Realisierung hat den Vorteil, dass auch schon bisher verwendete Bestimmungssysteme und/oder Trainingssysteme auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Eine Röntgenprojektion ist eine zweidimensionale Projektion eines Untersuchungsvolumens mittels Röntgenstrahlen entlang einer Projektionsrichtung, die insbesondere mehrere Pixel umfassen kann. Dabei wird jedem Pixel ein Röntgenintensitätswert zugewiesen, der ein Maß für die in diesem Pixel aufgetroffene Röntgenintensität ist. Die auftreffende Röntgenintensität hängt von der Zahl, der Größe, der Form und dem Material der sich im Untersuchungsvolumen befindlichen Objekte ab.

Ein zweidimensionaler Realbilddatensatz umfasst eine Röntgenprojektion, ein zweidimensionaler Realbilddatensatz kann insbesondere auch noch weitere Daten umfassen, insbesondere Metadaten betreffend die Röntgenprojektion (beispielsweise Zeitpunkt der Aufnahme der Röntgenprojektion, Projektionsrichtung der Röntgenprojektion, für die Röntgenprojektion verwendeter Röntgenstrom oder verwendete Röntgenspannung, persönliche Daten eines untersuchten Patienten, usw.). Insbesondere kann ein zweidimensionaler Realbilddatensatz auch identisch mit einer Röntgenprojektion sein.

Ein zweidimensionaler Differenzbilddatensatz eines Untersuchungsvolumens kann aus einer ersten Röntgenprojektion und einer zweiten Röntgenprojektion des Untersuchungsvolumens bestimmt werden, wobei die erste Röntgenprojektion und die zweite Röntgenprojektion bezüglich der gleichen Projektionsrichtung aufgenommen wurden, und wobei zum Zeitpunkt der Aufnahme der ersten Röntgenprojektion eine andere Kontrastmittelverteilung im Untersuchungsvolumen als zum Zeitpunkt der Aufnahme der zweiten Röntgenprojektion vorgelegen hat. Der zweidimensionale Differenzbilddatensatz kann aus der Subtraktion der Röntgenintensitäten der ersten Röntgenprojektion und der zweiten Röntgenprojektion berechnet werden. Ein so bestimmter zweidimensionaler Differenzbilddatensatz kann auch als zweidimensionaler Subtraktionsdatensatz bestimmt werden. Ein zweidimensionaler Differenzbilddatensatz kann auch mittels anderer Methoden bestimmt werden, beispielsweise durch Anwendung einer trainierten Funktion.

Aus mehreren zweidimensionalen Realbilddatensätzen oder aus mehreren zweidimensionalen Differenzbilddatensätzen, jeweils bezüglich unterschiedlicher Projektionsrichtungen, kann ein dreidimensionaler Bilddatensatz des Untersuchungsvolumens rekonstruiert werden. Insbesondere kann aus mehreren zweidimensionalen Realbilddatensätzen ein dreidimensionaler Realbilddatensatz rekonstruiert werden. Insbesondere kann aus mehreren zweidimensionalen Differenzbilddatensätzen ein dreidimensionaler Differenzbilddatensatz rekonstruiert werden. Ein dreidimensionaler Realbilddatensatz oder ein dreidimensionaler Differenzbilddatensatz kann insbesondere mehrere Voxel umfassen, denen eine Röntgenabsorption oder eine Röntgenintensität zugeordnet ist. Die Röntgenabsorption kann in Hounsfield-Einheiten (ein englischer Fachbegriff ist "Hounsfield-Units", kurz "HU") gemessen werden.

Im Allgemeinen bezeichnet eine Rekonstruktion das Bestimmen eines n-dimensionalen Bilddatensatzes basierend auf mehreren m-dimensionalen Bilddatensätzen, wobei m < n. Hierbei sind die mehreren m-dimensionalen Bilddatensätze insbesondere Projektionen eines n-dimensionalen Volumens, welches durch den n-dimensionalen Bilddatensatz beschrieben werden soll. Insbesondere kann eine Rekonstruktion das bestimmen eines dreidimensionalen Bilddatensatzes basierend auf mehreren zweidimensionalen Bilddatensätzen bezeichnen. Eine solche Rekonstruktion kann beispielsweise auf einer gefilterten Rückprojektion (ein englischer Fachbegriff ist "filtered back projection") basieren, alternativ sind dem Fachmann iterative Rekonstruktionsverfahren bekannt.

Ein vierdimensionaler Differenzbilddatensatz kann mehrere dreidimensionale Voxel umfassen, denen eine Zeitinformation zugeordnet ist. Äquivalent kann ein vierdimensionaler Differenzbilddatensatz auch dadurch beschrieben werden, dass er mehrere dreidimensionale Differenzbilddatensätze umfasst, wobei einem dreidimensionalen Differenzbilddatensatz eine Zeitinformation zugeordnet ist. Eine Zeitinformation kann als Zeitkoordinate aufgefasst werden, und der vierdimensionale Differenzbilddatensatz kann als zeitliche Abfolge oder Film von dreidimensionalen Differenzbilddatensätzen aufgefasst werden.

Eine Rückprojektion ist ein Verfahren, das aus einem oder mehreren zweidimensionalen Projektionen eines dreidimensionalen Untersuchungsvolumens Daten betreffend das dreidimensionale Untersuchungsvolumen ermittelt. Bei den Daten betreffend das dreidimensionale Untersuchungsvolumen kann es sich insbesondere um Absorptionskoeffizienten oder Hounsfield-Einheiten handeln. Da eine zweidimensionale Projektion weniger Informationen enthält als das dreidimensionale Untersuchungsvolumen, können für eine Rückprojektion weitere Informationen verwendet werden, beispielsweise eine Segmentierung des Untersuchungsvolumens oder eines Rekonstruktionsvolumens.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:

Fig. 1     ein Untersuchungsvolumen mit Gefäßen und einen dreidimensionalen Differenzbilddatensatz,
Fig. 2     zweidimensionale Realbilddatensätze des Untersuchungsvolumens,
Fig. 3     zweidimensionale Differenzbilddatensätze des Untersuchungsvolumens,
Fig. 4     schematisch ein Ausführungsbeispiel der Abhängigkeiten der verschiedenen Datensätze in den erfindungsgemäßen Verfahren,
Fig. 5     ein Ausführungsbeispiel des Verfahrens zum Bestimmen eines ersten Differenzbilddatensatzes,
Fig. 6     ein weiteres Ausführungsbeispiel des Verfahrens zum Bestimmen eines ersten Differenzbilddatensatzes,
Fig. 7     ein weiteres Ausführungsbeispiel des Verfahrens zum Bestimmen eines ersten Differenzbilddatensatzes,
Fig. 8     ein weiteres Ausführungsbeispiel des Verfahrens zum Bestimmen eines ersten Differenzbilddatensatzes, wobei weiterhin ein zweiter Differenzbilddatensatz bestimmt wird,
Fig. 9     eine erste trainierte Funktion,
Fig. 10    ein Ausführungsbeispiel des Verfahrens zum Anpassen einer ersten trainierten Funktion,
Fig. 11    ein weiteres Ausführungsbeispiel des Verfahrens zum Anpassen einer ersten trainierten Funktion,
Fig. 12    ein weiteres Ausführungsbeispiel des Verfahrens zum Anpassen einer ersten trainierten Funktion,
Fig. 13    ein Ausführungsbeispiel des Verfahrens zum Anpassen einer zweiten trainierten Funktion,
Fig. 14    ein Bestimmungssystem und ein Trainingssystem,
Fig. 15    eine Röntgeneinheit,
Fig. 16    einen dreidimensionalen Differenzbilddatensatz und einen Vergleichsbilddatensatz.

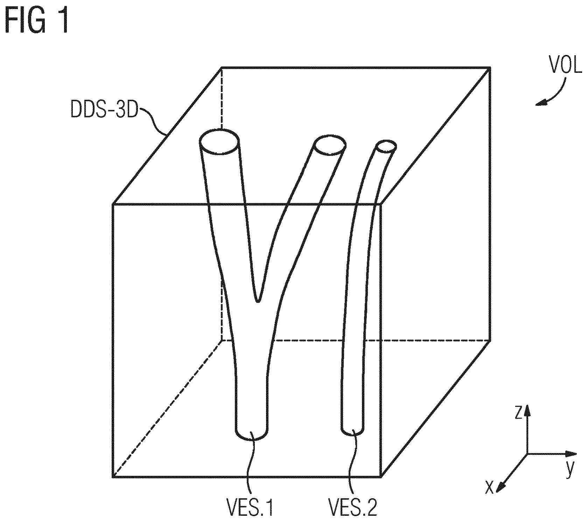

Fig. 1 zeigt ein Untersuchungsvolumen VOL mit zwei Gefäßen VES.1, VES.2, sowie einen dreidimensionalen

EP 3 591 617 A1

ersten Differenzbilddatensatz DDS-3D. Hierbei entspricht der Bildbereich des dreidimensionalen ersten Differenzbilddatensatzes DDS-3D dem Untersuchungsvolumen VOL. Im dargestellten Ausführungsbeispiel umfasst das Untersuchungsvolumen ein erstes Gefäß VES.1 und ein zweites Gefäß VES.2, wobei sich das erste Gefäß VES.1 innerhalb des Untersuchungsvolumens VOL in zwei Äste verzweigt. Es ist auch möglich, dass das Untersuchungsvolumen kein Gefäß VES.1, VES.2, genau ein Gefäß VES.1, VES.2 oder mehr als zwei Gefäße VES.1, VES.2 umfasst. Das Untersuchungsvolumen VOL umfasst neben den Gefäßen VES.1, VES.2 weitere Strukturen OS.1, OS.2, die im dreidimensionalen ersten Differenzbilddatensatz DDS-3D nicht abgebildet sind, da diese dem Hintergrund zuzurechnen sind, und daher in dem dreidimensionalen ersten Differenzbilddatensatz nicht abgebildet werden.

Im dargestellten Ausführungsbeispiel sind das Untersuchungsvolumen VOL sowie der dreidimensionale erste Differenzbilddatensatz DDS-3D bezüglich einer ersten Richtung x, einer zweiten Richtung y und einer dritten Richtung z ausgedehnt. Die erste Richtung x, die zweite Richtung y und die dritte Richtung z sind hierbei paarweise orthogonal.

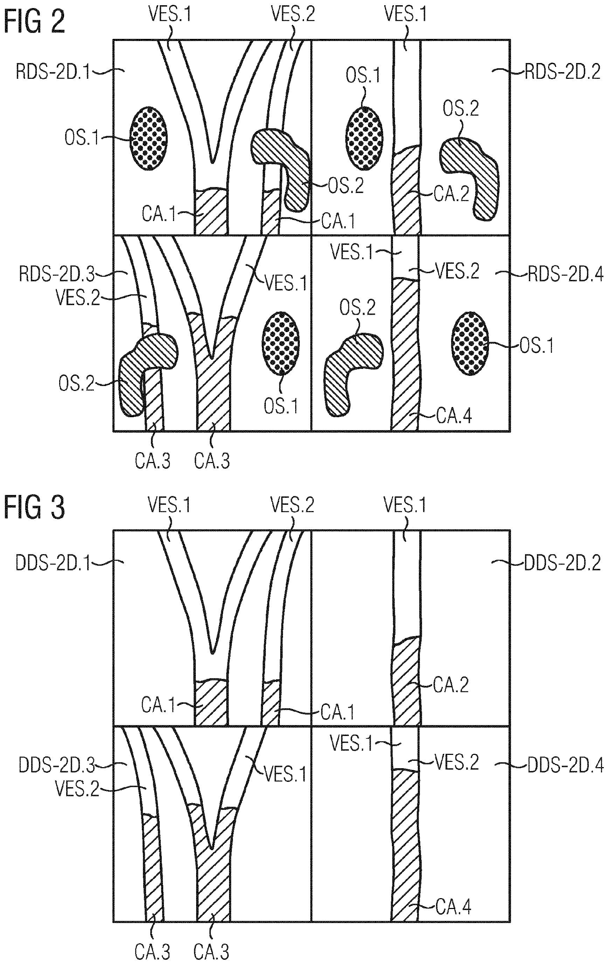

Fig. 2 zeigt mehrere zweidimensionale Realbilddatensätze RDS-2D.1, ..., RDS-2D.4 des Untersuchungsvolumens VOL, Fig. 3 zeigt mehrere zweidimensionale Differenzbilddatensätze DDS-2D.1, ..., DDS-2D.4 des Untersuchungsvolumens VOL. Im dargestellten Ausführungsbeispiel sind vier zweidimensionale Realbilddatensätze RDS-2D.1, ..., RDS-2D.4 dargestellt, es können auch mehr oder weniger zweidimensionale Realbilddatensätze RDS-2D.1, ..., RDS-2D.4 vorhanden sein bzw. verwendet werden. Weiterhin sind vier zweidimensionale Differenzbilddatensätze DDS-2D.1, ..., DDS-2D.4 dargestellt, es können aber auch mehr oder weniger zweidimensionale Differenzbilddatensätze DDS-2D.1, ..., DDS-2D.4 vorhanden sein bzw. verwendet werden.

Jeder der zweidimensionalen Realbilddatensätze RDS-2D.1, ..., RDS-2D.4 umfasst hierbei eine Röntgenprojektion des Untersuchungsvolumens VOL bezüglich einer Projektionsrichtung. Der zweidimensionale Realbilddatensatz RDS-2D.1 umfasst eine Röntgenprojektion des Untersuchungsvolumens VOL bezüglich einer Projektionsrichtung, wobei die Projektionsrichtung antiparallel zur ersten Richtung x ist. Der zweidimensionale Realbilddatensatz RDS-2D.2 umfasst eine Röntgenprojektion des Untersuchungsvolumens VOL bezüglich einer Projektionsrichtung, wobei die Projektionsrichtung antiparallel zur zweiten Richtung y ist. Der zweidimensionale Realbilddatensatz RDS-2D.3 umfasst eine Röntgenprojektion des Untersuchungsvolumens VOL bezüglich einer Projektionsrichtung, wobei die Projektionsrichtung parallel zur ersten Richtung x ist. Der zweidimensionale Realbilddatensatz RDS-2D.4 umfasst eine Röntgenprojektion des Untersuchungsvolumens VOL bezüglich einer Projektionsrichtung, wobei die Projektionsrichtung parallel zur zweiten Richtung y ist.

Im dargestellten Ausführungsbeispiel korrespondiert jeder der zweidimensionalen Differenzbilddatensätze DDS-2D.1, ..., DDS-2D.4 mit einem der zweidimensionalen Realbilddatensätze RDS-2D.1, ..., RDS-2D.4. Beispielsweise korrespondiert der zweidimensionale Differenzbilddatensatz DDS-2D.1 mit dem zweidimensionalen Realbilddatensatz RDS-2D.1, der zweidimensionale Differenzbilddatensatz DDS-2D.2 korrespondiert mit dem zweidimensionalen Realbilddatensatz RDS-2D.2, der zweidimensionale Differenzbilddatensatz DDS-2D.3 korrespondiert mit dem zweidimensionalen Realbilddatensatz RDS-2D.3, und der zweidimensionale Differenzbilddatensatz DDS-2D.4 korrespondiert mit dem zweidimensionalen Realbilddatensatz RDS-2D.4.

Insbesondere umfasst jeder der zweidimensionalen Differenzbilddatensätze DDS-2D.1, ..., DDS-2D.4 Differenzen von Intensitäten zwischen einem der zweidimensionalen Realbilddatensätze RDS-2D.1, ..., RDS-2D.4 und einem weiteren zweidimensionalen Realbilddatensatz, insbesondere einer Maskenaufnahme. Beispielsweise umfasst der zweidimensionale Differenzbilddatensatz DDS-2D.1 Differenzen von Intensitäten des zweidimensionalen Realbilddatensatzes RDS-2D.1 und einem weiteren zweidimensionalen Realbilddatensatz, insbesondere einer Maskenaufnahme, der zweidimensionale Differenzbilddatensatz DDS-2D.2 umfasst Differenzen von Intensitäten des zweidimensionalen Realbilddatensatzes RDS-2D.3 und einem weiteren zweidimensionalen Realbilddatensatz, insbesondere einer Maskenaufnahme, der zweidimensionale Differenzbilddatensatz DDS-2D.3 umfasst Differenzen von Intensitäten des zweidimensionalen Realbilddatensatzes RDS-2D.2 und einem weiteren zweidimensionalen Realbilddatensatz, insbesondere einer Maskenaufnahme und der zweidimensionale Differenzbilddatensatz DDS-2D.4 umfasst Differenzen von Intensitäten des zweidimensionalen Realbilddatensatzes RDS-2D.4 und einem weiteren zweidimensionalen Realbilddatensatz, insbesondere einer Maskenaufnahme.

Weiterhin ist jedem der zweidimensionalen Realbilddatensätze RDS-2D.1, ..., RDS-2D.4 ein Zeitpunkt zugeordnet, wobei dieser Zeitpunkt in diesem Ausführungsbeispiel dem Zeitpunkt der Aufnahme der zugehörigen Röntgenprojektion entspricht. Insbesondere ist also dem zweidimensionalen Realbilddatensatz RDS-2D.1 der Zeitpunkt $t_1$ der Aufnahme der zugehörigen Röntgenprojektion zugeordnet. Insbesondere ist also dem zweidimensionalen Realbilddatensatz RDS-2D.2 der Zeitpunkt $t_2$ der Aufnahme der zugehörigen Röntgenprojektion zugeordnet. Insbesondere ist also dem zweidimensionalen Realbilddatensatz RDS-2D.3 der Zeitpunkt $t_3$ der Aufnahme der zugehörigen Röntgenprojektion zugeordnet. Insbesondere ist also dem zweidimensionalen Realbilddatensatz RDS-2D.4 der Zeitpunkt $t_4$ der Aufnahme der zugehörigen Röntgenprojektion zugeordnet.

Im dargestellten Ausführungsbeispiel sind auch den zweidimensionalen Differenzbilddatensätzen DDS-2D.1, ..., DDS-2D.4 jeweils ein Zeitpunkt zugeordnet, insbesondere ist jedem der zweidimensionalen Differenzbilddatensätze DDS-2D.1, ..., DDS-2D.4 der Zeitpunkt des korrespondierenden zweidimensionalen Realbilddatensatzes RDS-2D.1,

..., RDS-2D.4 zugeordnet.

Im dargestellten Ausführungsbeispiel bildet jeder der zweidimensionalen Realbilddatensätze RDS-2D.1, ..., RDS-2D.4 bzw. die zugeordnete Röntgenprojektion die im Untersuchungsvolumen VOL enthaltenen Gefäße VES.1, VES.2 ab. Weiterhin werden von den zweidimensionalen Realbilddatensätzen RDS-2D.1, ..., RDS-2D.4 sonstige Strukturen OS.1, OS.2 abgebildet.

Zu den Zeitpunkten $t_1$, ..., $t_4$ der Aufnahme der zweidimensionalen Realbilddatensätze RDS-2D.1, ..., RDS-2D.4 umfassen die Gefäße VES.1, VES.2 zeitlich unterschiedliche Konzentrationen CA.1, ..., CA.4 von Kontrastmittel. Hierbei ist dem Zeitpunkt $t_1$ die Konzentration CA.1 zugeordnet, dem Zeitpunkt $t_2$ ist die Konzentration CA.2 zugeordnet, dem Zeitpunkt $t_3$ ist die Konzentration CA.3 zugeordnet, und dem Zeitpunkt $t_4$ ist die Konzentration CA.4 zugeordnet. Bei dem Kontrastmittel handelt es sich hierbei um ein Röntgenkontrastmittel, so dass die jeweilige Kontrastmittelkonzentration CA.1, ..., CA.4 des Kontrastmittels aus den Röntgenprojektionen bestimmbar ist. Die Kontrastmittelkonzentration CA.1, ..., CA.4 ändert sich zeitlich durch eine statische oder dynamische Flüssigkeitsströmung in den Gefäßen VES.1, VES.2. Im dargestellten Ausführungsbeispiel handelt es sich bei der Flüssigkeit um Blut.

Im Ausführungsbeispiel umfassen die zweidimensionalen Differenzbilddatensätze DDS-2D.1, ..., DDS-2D.4 Differenzen von Intensitäten der zweidimensionalen Realbilddatensätze RDS-2D.1, ..., RDS-2D-4 und jeweiligen Maskenaufnahmen. In diesem Ausführungsbeispiel sind die Maskenaufnahmen Röntgenprojektionen des Untersuchungsvolumens VOL bezüglich einer Projektionsrichtung, wobei die Projektionsrichtung der Projektionsrichtung der Röntgenprojektion des jeweiligen zweidimensionalen Realbilddatensatzes RDS-2D.1, ..., RDS-2D.4 entspricht, und wobei zum Zeitpunkt der Maskenaufnahme kein Kontrastmittel im Gefäß VES.1, VES.2 vorhanden ist. Weiterhin wird davon ausgegangen, dass die Abbildungsgeometrie (insbesondere die relativen Positionen und die relativen Orientierungen von Untersuchungsvolumen VOL, Röntgenquelle XRAY.SRC und Röntgendetektor XRAY.DTC) des zweidimensionalen Realbilddatensatzes RDS-2D.1, ..., RDS-2D.4 und der zugehörigen Maskenaufnahme identisch sind, und dass bis auf die Änderung der Kontrastmittelkonzentration CA.1, ..., CA.4 keine Änderungen oder Bewegungen im Untersuchungsvolumen VOL vorliegen. Abweichungen der Projektionsgeometrie, Änderungen oder Bewegungen im Untersuchungsvolumen VOL sind möglich, können dann aber zu Bildartefakten führen. Die zweidimensionalen Differenzbilddatensätze DDS-2D.1, ..., DDS-2D.4 können aus einer Subtraktion eines zweidimensionalen Realbilddatensatzes RDS-2D.1, ..., RDS-2D-4 und der jeweiligen Maskenaufnahme bestimmt werden, alternativ können die die zweidimensionalen Differenzbilddatensätze DDS-2D.1, ..., DDS-2D.4 auch durch Anwendung einer ersten trainierten Funktion TF-2 auf die zweidimensionalen Realbilddatensätze RDS-2D.1, ..., RDS-2D.4 bestimmt werden, ohne dass die Röntgenprojektionen der Maskenaufnahmen bekannt sind bzw. tatsächlich aufgenommen wurden.

Im dargestellten Ausführungsbeispiel sind durch diese Festlegungen die sonstigen Strukturen OS.1, OS.2 der zweidimensionalen Realbilddatensätze RDS-2D.1, ..., RDS-2D.4 in den zweidimensionalen Differenzbilddatensätzen DDS-2D.1, ..., DDS-2D.4 unterdrückt bzw. nicht enthalten bzw. nicht dargestellt. Weiterhin können die Ränder der Gefäße VES.1, VES.2, die in der Fig. 3 schematisch dargestellt sind, nicht in den zweidimensionalen Differenzbilddatensätzen DDS-2D.1, ..., DDS-2D.4 enthalten sein bzw. unterdrückt sein. Insbesondere können die zweidimensionalen Differenzbilddatensätze DDS-2D.1, ..., DDS-2D.4 nur das Kontrastmittel bzw. die Kontrastmittelkonzentration CA.1, ..., CA.4 darstellen, da (bis auf Artefakte) im Untersuchungsvolumen VOL nur die Kontrastmittelkonzentration CA.1, ..., CA.4 veränderlich ist.

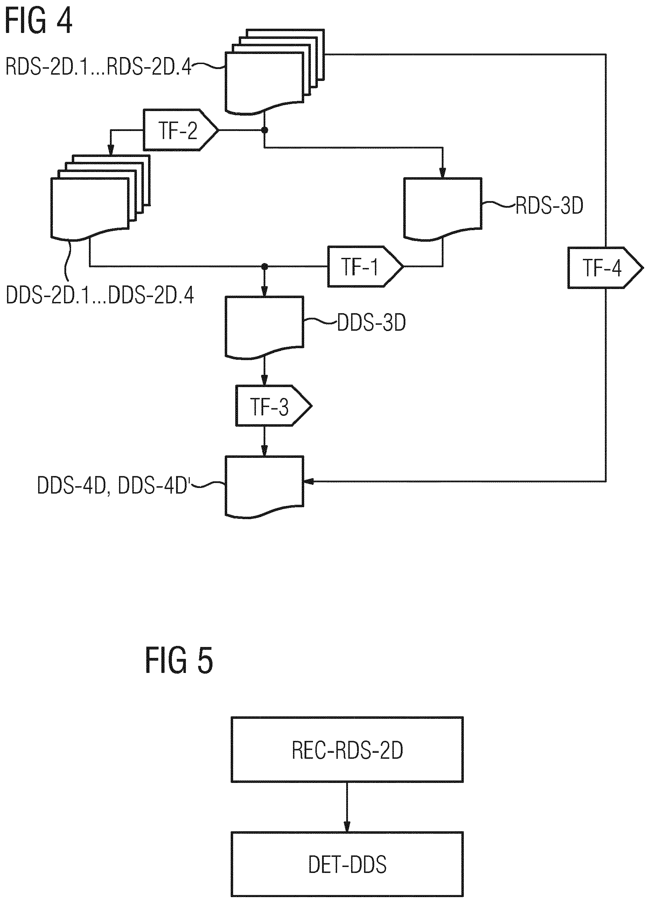

Fig. 4 zeigt schematisch ein Ausführungsbeispiel der Abhängigkeiten der verschiedenen Datensätze, die als Eingabewerte, Ausgabewerte oder Zwischenergebnisse eines Verfahrens zum Bestimmen eines ersten Differenzbilddatensatzes verwendet werden können.

Ausgangspunkt des Verfahrens zum Bestimmen eines ersten Differenzbilddatensatzes sind jeweils zweidimensionalen Realbilddatensätze RDS-2D.1, ..., RDS-2D.4 eines Untersuchungsvolumens VOL.

Basierend auf den zweidimensionalen Realbilddatensätzen RDS-2D.1, ..., RDS-2D.4 kann, beispielsweise durch bekannte Rekonstruktionsalgorithmen, ein dreidimensionaler Realbilddatensatz RDS-3D des Untersuchungsvolumens VOL bestimmt werden. Ausgehend vom dreidimensionalen Realbilddatensatz RDS-3D kann dann, beispielsweise durch Anwendung einer ersten trainierten Funktion TF-1 auf den dreidimensionalen Realbilddatensatz RDS-3D, ein dreidimensionaler erster Differenzbilddatensatz DDS-3D bestimmt werden.

Alternativ können, insbesondere durch Anwendung einer ersten trainierten Funktion TF-2 oder durch Subtraktion einer Maskenaufnahmen, basierend auf den zweidimensionalen Realbilddatensätze RDS-2D.1, ..., RDS-2D.4 zweidimensionale Differenzbilddatensätze DDS-2D.1, ..., DDS-2D.4 bestimmt werden. Mittels bekannter Rekonstruktionsalgorithmen kann dann der dreidimensionale erste Differenzbilddatensatz DDS-3D basierend auf den zweidimensionalen Differenzbilddatensätzen DDS-2D.1, ..., DDS-2D.4 bestimmt werden.

Auf Grundlage des dreidimensionalen ersten Differenzbilddatensatzes DDS-3D kann dann ein vierdimensionaler zweiter Differenzbilddatensatz DDS-4D' bestimmt werden. Dieses Bestimmen des vierdimensionalen zweiten Differenzbilddatensatzes DDS-4D' kann weiterhin auf den zweidimensionalen Realbilddatensätzen RDS-2D.1, ..., RDS-2D.4 und/oder den zweidimensionalen Differenzbilddatensätzen DDS-2D.1, ..., DDS-2D.4 basieren. Beispielsweise können die zweidimensionalen Realbilddatensätze RDS-2D.1, ..., RDS-2D.4 und/oder die zweidimensionalen Differenzbilddatensätze DDS-2D.1, ..., DDS-2D.4 auf den dreidimensionalen ersten Differenzbilddatensatz DDS-3D oder eine Segmentierung des dreidimensionalen ersten Differenzbilddatensatzes DDS-3D zurückprojiziert werden.

Alternativ kann, insbesondere durch Anwendung einer ersten Trainierten Funktion TF-3, basierend auf den zweidimensionalen Realbilddatensätzen RDS-2D.1, ..., RDS-2D.4 ein vierdimensionaler erster Differenzbilddatensatz DDS-4D bestimmt werden. In diesem Fall entfällt der Zwischenschritt der Berechnung eines dreidimensionalen Differenzbilddatensatzes.

Fig. 5 zeigt ein erstes Ausführungsbeispiel des Verfahrens zum Bestimmen eines ersten Differenzbilddatensatzes DDS-3D, DDS-4D eines Untersuchungsvolumens VOL, wobei der erste Differenzbilddatensatz DDS-2D.1, ..., DDS-2D.4, DDS-3D, DDS-4D mindestens zweidimensional ist, insbesondere mindestens dreidimensional ist. In diesem Ausführungsbeispiel ist der erste Differenzbilddatensatz DDS-3D, DDS-4D dreidimensional oder vierdimensional.

Der erste Schritt des ersten Ausführungsbeispiels ist das Empfangen REC-RDS-2D von zweidimensionalen Realbilddatensätzen RDS-2D.1, ..., RDS-2D.4 betreffend ein Untersuchungsvolumen VOL mittels einer Schnittstelle SYS.IF, wobei jeder der zweidimensionalen Realbilddatensätze RDS-2D.1, ..., RDS-2D.4 eine zweidimensionale Röntgenprojektion des Untersuchungsvolumens VOL bezüglich einer Projektionsrichtung umfasst.

Insbesondere können die zweidimensionalen Realbilddatensätze RDS-2D.1, ..., RDS-2D.4 vorab mittels einer Röntgeneinrichtung XRAY aufgenommen werden. Insbesondere können hierbei eine Röntgenquelle XRAY.SRC und/oder ein Röntgendetektor XRAY.DTC um das Untersuchungsvolumen VOL rotieren. Insbesondere können die Röntgenquelle XRAY.SRC und der Röntgendetektor XRAY.DTC simultan um das Untersuchungsvolumen rotieren. Bei einer Röntgeneinrichtung XRAY kann es sich insbesondere um ein C-Bogen-Röntgengerät handeln.

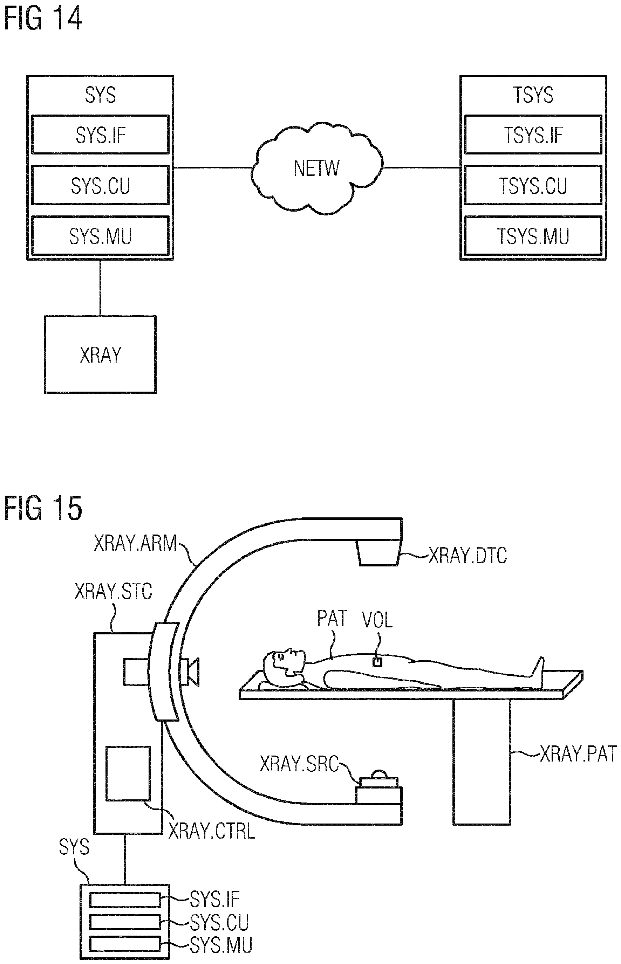

Im dargestellten Ausführungsbeispiel wurden die Röntgenprojektionen der zweidimensionalen Realbilddatensätze RDS-2D.1, ..., RDS-2D.4 mit einem C-Bogen-Röntgengerät aufgenommen (ein solches C-Bogen-Röntgengerät ist in Fig. 15 dargestellt). Dabei werden die zweidimensionalen Realbilddatensätze RDS-2D.1, ..., RDS-2D.4 derart aufgenommen, dass der C-Bogen XRAY.ARM einen vorgegebenen Winkel um das Untersuchungsvolumen VOL rotiert und im konstanten zeitlichen Abstand Röntgenprojektionen aufgenommen werden. Werden optionale Maskenaufnahmen durchgeführt, können diese Maskenaufnahmen mit den gleichen Projektionsrichtungen ebenfalls mit der beschriebenen Aufnahmetechnik aufgenommen werden.

Bei jeder Aufnahmeserie rotiert der C-Bogen XRAY.ARM des C-Bogen-Röntgengeräts XRAY in diesem Ausführungsbeispiel in 12 Sekunden um 260° und nimmt dabei 304 zweidimensionale Realbilddatensätze RDS-2D.1, ..., RDS-2D.4 aus unterschiedlichen Projektionsrichtungen auf. Es sind auch Aufnahmeparameter umfassend andere Rotationswinkel, Rotationszeiträume und Projektionszahlen möglich, insbesondere solche Aufnahmeparameter, die zu zweidimensionalen Realbilddatensätze RDS-2D.1, ..., RDS-2D.4 führen, die zu einer dreidimensionalen Rekonstruktion geeignet sind. Hierbei sind insbesondere Rotationswinkel geeignet, die größer sind als die Summe von 180° und dem Öffnungswinkel der Röntgenstrahlen der Röntgenquelle XRAY.SRC, insbesondere Rotationswinkel größer als 200°.

Im Folgenden wird die Projektionsrichtung einer Röntgenprojektion eines der zweidimensionalen Realbilddatensätze RDS-2D.1, ..., RDS-2D.4 mit $v_i$ bezeichnet, wobei die Projektionsrichtung hierbei ein dreidimensionaler Vektor ist, insbesondere ein dreidimensionaler Einheitsvektor. Im diesem ersten Ausführungsbeispiel umfasst jeder der zweidimensionalen Realbilddatensätze RDS-2D.1, ..., RDS-2D.4 weiterhin einen Zeitpunkt $t_i$ der Aufnahme der jeweiligen Röntgenprojektion.

Näherungsweise ist dann der folgende Zusammenhang gültig:

$$b_{kl}(t_i) = \log\left(\frac{I_{kl}(t_i)}{I_0}\right) = -\int_{\Gamma_{kl}(v_i)} \mu(x, t_i)dx$$

Hierbei ist $I_0$ die Röntgenintensität der Röntgenquelle, $I_{kl}(t_i)$ ist die Röntgenintensität im Pixel mit den Koordinaten k, i im Röntgendetektor XRAY.DTC bzw. im zweidimensionalen Realbilddatensatz RDS-2D.1, ..., RDS-2D.4 zum Zeitpunkt $t_i$. $\Gamma_{kl}(v_i)$ ist der Pfad von der Röntgenquelle XRAY.SRC zum Pixel mit den Koordinaten k, i im Röntgendetektor XRAY.DTC bzw. im zweidimensionalen Realbilddatensatz RDS-2D.1, ..., RDS-2D.4, wenn die Projektionsrichtung $v_i$ entspricht (näherungsweise kann hier eine Parallelprojektion angenommen werden), und $\mu(x,t_i)$ ist der lineare Abschwächungskoeffizient an der Stelle bzw. dreidimensionalen Koordinate x des Untersuchungsvolumens zum Zeitpunkt $t_i$. Der Pfad $\Gamma_{kl}(v_i)$ kann hierbei durch einfache geometrische Überlegungen bestimmt werden, weiterhin kann der Zusammenhang auch kontinuierlich beschrieben werden:

$$b(y, t_i) = \log\left(\frac{I(y, t_i)}{I_0}\right) = -\int_{\Gamma(y, v_i)} \mu(x, t_i)dx$$

Hierbei ist dann I(y, $t_i$) die Röntgenintensität in der zweidimensionalen Koordinate y im Röntgendetektor XRAY.DTC bzw. im zweidimensionalen Realbilddatensatz RDS-2D.1, ..., RDS-2D.4 zum Zeitpunkt $t_i$, und $\Gamma$(y, $v_i$) ist der Pfad von der Röntgenquelle XRAY.SRC zum Punkt mit den zweidimensionalen Koordinaten y bezüglich des Röntgendetektors XRAY.DTC bzw. bezüglich des zweidimensionalen Realbilddatensatzes RDS-2D.1, ..., RDS-2D.4, wenn die Projektionsrichtung $v_i$ entspricht.

Im dargestellten Ausführungsbeispiel umfassen die zweidimensionalen Realbilddatensätze RDS-2D.1, ..., RDS-2D.4 den Logarithmus $b_{kl}(t_i)$ = log($I_{kl}(t_i)$ / $I_0$) der relativen Röntgenintensitäten $I_{kl}(t_i)$ / $I_0$, alternativ können die zweidimensionalen Realbilddatensätze RDS-2D.1, ..., RDS-2D.4 auch die Röntgenintensitäten $I_{kl}(t_i)$ direkt umfassen. Beide Alternativen können, bei Kenntnis der Intensität $I_0$ der Röntgenquelle XRAY.SRC, ineinander umgerechnet werden.

Der zweite Schritt des ersten Ausführungsbeispiels ist das Bestimmen DET-DDS des ersten Differenzbilddatensatzes DDS-3D, DET-DDS basierend auf den zweidimensionalen Realbilddatensätzen RDS-2D.1, ..., RDS-2D.4 und basierend auf einer trainierten Funktion TF-1, TF-2, TF-3 mittels einer Recheneinheit SYS.CU. Das Bestimmen DET-DDS kann hierbei insbesondere nur auf denjenigen zweidimensionalen Realbilddatensätzen RDS-2D.1, ..., RDS-2D.4 basieren, bei denen das Gefäß VES.1, VES.2 komplett, oder zu einem hohen Anteil (insbesondere zu mehr als 75%, insbesondere zu mehr als 90%) mit Kontrastmittel gefüllt ist.

In diesem Ausführungsbeispiel umfasst die erste trainierte Funktion TF-1, TF-2, TF-3 wenigstens eine Faltungsschicht (ein englischer Fachbegriff ist "convolutional layer" und eine Pooling-Schicht (ein englischer Fachbegriff ist "pooling layer"). Insbesondere umfasst die erste trainierte Funktion TF-1, TF-2, TF-3 ein faltendes neuronales Netzwerk (ein englischer Fachbegriff ist "convolutional neural network", kurz "CNN"), wobei der dreidimensionale Realbilddatensatz als Eingabewert des faltenden neuronalen Netzwerks verwendet wird. Insbesondere kann die erste trainierte Funktion TF-1, TF-2, TF-3 ein volles faltendes neuronales Netzwerk (ein englischer Fachbegriff ist "fully convolutional neural network", kurz "FCNN") sein, wobei ein FCNN ein CNN ist, wobei die letzte vollständig verbundene Schicht des CNN durch eine Faltungsschicht und/oder Entfaltungsschicht ersetzt wird.

Insbesondere kann in diesem ersten Ausführungsbeispiel der erste Differenzbilddatensatz DDS-4D vierdimensional sein, und der vierdimensionale erste Differenzbilddatensatz DDS-4d wird durch Anwendung der ersten trainierten Funktion TF-3 auf die zweidimensionalen Realbilddatensätze RDS-2D.1, ..., RDS-2D.4 berechnet. Hierbei ist die erste trainierte Funktion TF-3 also insbesondere eine Funktion, die mehrere zweidimensionale Bilddatensätze auf einen vierdimensionalen Bilddatensatz abbildet.

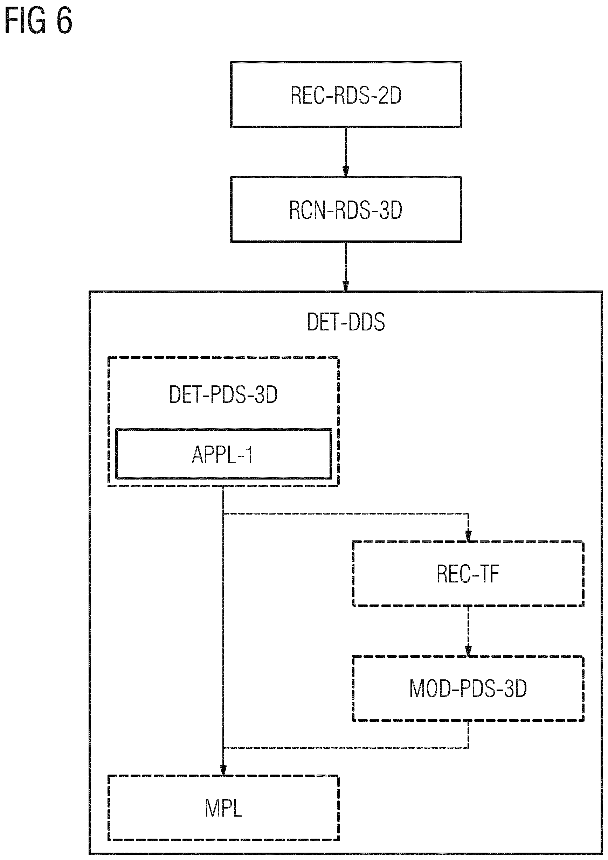

Fig. 6 zeigt ein zweites Ausführungsbeispiel des Verfahrens zum Bestimmen eines ersten Differenzbilddatensatzes DDS-3D eines Untersuchungsvolumens VOL, wobei in diesem Ausführungsbeispiel der erste Differenzbilddatensatz DDS-3D dreidimensional ist. Das zweite Ausführungsbeispiel umfasst die Schritte des Empfangens REC-RDS-2D von zweidimensionalen Realbilddatensätzen RDS-2D.1, ..., RDS-2D.4 sowie das Bestimmen DET-DDS des ersten Differenzbilddatensatzes DDS-3D, diese Schritte können alle vorteilhaften Aus- und Weiterbildungen der entsprechenden Schritte des ersten Ausführungsbeispiels aufweisen.

Das zweite Ausführungsbeispiel umfasst weiterhin das Rekonstruieren RCN-RDS-3D eines dreidimensionalen Realbilddatensatzes RDS-3D basierend auf den zweidimensionalen Realbilddatensätzen RDS-2D.1, ..., RDS-2D.4 mittels der Recheneinheit SYS.CU. In diesem Ausführungsbeispiel wird der dreidimensionale Realbilddatensatz RDS-3D durch Anwendung der gefilterten Rückprojektion bestimmt, alternativ können auch andere Rekonstruktionsverfahren, insbesondere iterative Rekonstruktionsverfahren verwendet werden.

Das Rekonstruieren RCN-RDS-3D des dreidimensionalen Realbilddatensatzes RDS-3D kann insbesondere nur auf den zweidimensionalen Bilddatensätzen RDS-2D.1, ..., RDS-2D.4 basieren, bei denen das Gefäß VES.1, VES.2 komplett oder nahezu komplett mit Kontrastmittel gefüllt ist. Alternativ kann das Rekonstruieren RCN-RDS-3D des dreidimensionalen Realbilddatensatzes RDS-3D auch auf allen zweidimensionalen Bilddatensätzen RDS-2D.1, ..., RDS-2D.4 basieren.

Die Intensitäten des dreidimensionalen Realbilddatensatzes RDS-3D können insbesondere den Röntgenabsorptionskoeffizienten bzw. den linearen Abschwächungskoeffizienten $\mu$ des Rekonstruktionsvolumens bzw. des Untersuchungsvolumens VOL entsprechen. Insbesondere kann der dreidimensionale Realbilddatensatz RDS-3D die Zeitabhängigkeit der linearen Abschwächungskoeffizienten berücksichtigen:

$$B(x) = \mu(x, t > t')$$

$$B(x) = \frac{1}{t'' - t'} \int_{t'}^{t''} \mu(x, t)dt$$

Hierbei entsprechen im ersten Fall die Intensitäten des dreidimensionalen Realbilddatensatzes RDS-3D den linearen Abschwächungskoeffizienten im Untersuchungsvolumen nach dem Zeitpunkt t', wobei der Zeitpunkt t' insbesondere dem Zeitpunkt entsprechen kann, zu dem das Gefäß vollständig mit Kontrastmittel gefüllt war. Im zweiten Fall entsprechen die Intensitäten des dreidimensionalen Realbilddatensatzes RDS-3D einer Mittelung der linearen Abschwächungskoeffizienten im Untersuchungsvolumen VOL über einen Zeitraum von t' bis t''. Der dreidimensionale Realbilddatensatz RDS-3D kann insbesondere auch durch diskrete Voxel definiert sein, wobei die Intensitäten der Voxel beispielsweise über eine räumliche Mittelung von B(x) über das Volumen des jeweiligen Voxels bestimmt werden können.

Alternativ können in gleicher Weise die Intensitäten des dreidimensionalen Realbilddatensatzes RDS-3D auch relativen linearen Abschwächungskoeffizienten $\mu$ des Rekonstruktionsvolumens bzw. des Untersuchungsvolumens VOL entsprechen, insbesondere Hounsfield-Einheiten (welche auf dem linearen Abschwächungskoeffizienten $\mu$ relativ zum linearen Abschwächungskoeffizienten von Wasser basieren).

Im dargestellten zweiten Ausführungsbeispiel umfasst das Bestimmen DET-DDS des dreidimensionalen ersten Differenzbilddatensatzes DDS-3D ein Bestimmen DET-PDS-3D eines dreidimensionalen Wahrscheinlichkeitsdatensatzes PDS-3D durch Anwenden APPL-1 der ersten trainierten Funktion TF-1 auf den dreidimensionalen Realbilddatensatz RDS-3D mittels der Recheneinheit SYS.CU sowie ein pixelweises Multiplizieren MPL des dreidimensionalen Wahrscheinlichkeitsdatensatzes PDS-3D mit dem dreidimensionalen Realbilddatensatz RDS-3D mittels der Recheneinheit SYS.CU

Optional umfasst im zweiten Ausführungsbeispiel das Bestimmen DET-DDS weiterhin das Empfangen REC-TF einer Transferfunktion mittels der Schnittstelle SYS.IF und das Modifizieren MOD-PDS-3D des dreidimensionalen Wahrscheinlichkeitsdatensatzes PDS-3D basierend auf einer Transferfunktion mittels der Recheneinheit SYS.CU. Insbesondere werden diese beiden Schritte nach dem Bestimmen DET-PDS-3D eines dreidimensionalen Wahrscheinlichkeitsdatensatzes PDS-3D und vor dem pixelweisen Multiplizieren MPL ausgeführt. In diesem Fall wird der dreidimensionale Realbilddatensatz RDS-3D mit dem modifizierten dreidimensionalen Wahrscheinlichkeitsdatensatz PDS-3D multipliziert.

In diesem Ausführungsbeispiel ist eine Transferfunktion T eine monoton steigende Funktion, die Wahrscheinlichkeitswerte im Intervall [0; 1] auf Wahrscheinlichkeitswerte im Intervall [0; 1] abbildet, wobei $T(0) = 0$ und $T(1) = 1$ gilt. Die Transferfunktion T wird hierbei pixelweise auf jeden Wahrscheinlichkeitswert des dreidimensionalen Wahrscheinlichkeitsdatensatzes angewendet. Beispiele für Transferfunktionen sind $T(x) = x^\gamma$ mit $\gamma > 0$ oder Rampenfunktionen. Durch Transferfunktionen können Wahrscheinlichkeitswerte insbesondere reskaliert werden, und durch eine geeignete Wahl der Transferfunktionen können Bildartefakte bzw. Artefakte in den Wahrscheinlichkeitswerten reduziert und/oder unterdrückt werden.

Bezeichnet $B_{klm}$ den dreidimensionalen Realbilddatensatz RDS-3D, $D_{klm}$ den dreidimensionalen Differenzbilddatensatz DDS-3D, und $W_{klm}$ den dreidimensionalen Wahrscheinlichkeitsdatensatzes PDS-3D, so wird also in diesem Ausführungsbeispiel der dreidimensionale erste Differenzbilddatensatz DDS-3D wie folgt bestimmt:

$$D_{klm} = W_{klm} \cdot B_{klm} = T(F_1(B_{111}, \dots, B_{k'l'm'}, \dots, B_{KLM})) \cdot B_{klm}$$

Hierbei ist $F_1$ die erste trainierte Funktion TF-1, und K,L und M sind die Ausdehnung sowohl des dreidimensionalen Realbilddatensatzes RDS-3D als auch des dreidimensionalen ersten Differenzbilddatensatzes DDS-3D bezüglich der ersten Richtung x, der zweiten Richtung y und der dritten Richtung z, gemessen jeweils in der Anzahl der Voxel. Das Argument der Funktion $F_1$ ist derart zu verstehen, dass der Intensitätswert $D_{klm}$ des dreidimensionalen ersten Differenzbilddatensatzes DDS-3D am Pixel mit den Indizes k, l und m von allen Intensitätswerten des dreidimensionalen Realbilddatensatzes RDS-3D abhängig sein kann.

Alternativ wird das Bestimmen DET-DDS des dreidimensionalen ersten Differenzbilddatensatzes DDS-3D dadurch ausgeführt, dass eine erste trainierte Funktion TF-1 als Eingabedaten den dreidimensionalen Realbilddatensatz RDS-3D erhält, und als Ausgabedaten den dreidimensionalen ersten Differenzbilddatensatz DDS-3D erzeugt. Dadurch umfasst das Bestimmen DET-DDS des dreidimensionalen ersten Differenzbilddatensatzes DDS-3D auch ein Anwenden APPL-1 der ersten trainierten Funktion TF-1 auf den dreidimensionalen Realbilddatensatz RDS-3D mittels der Recheneinheit SYS.CU.

Bezeichnet $B_{klm}$ den dreidimensionalen Realbilddatensatz RDS-3D, und $D_{klm}$ den dreidimensionalen Differenzbilddatensatz DDS-3D, so wird also in dieser Alternative der dreidimensionale erste Differenzbilddatensatz DDS-3D wie folgt bestimmt:

$$D_{klm} = F_1(B_{111}, \dots, B_{k'l'm'}, \dots, B_{KLM})$$

Hierbei ist $F_1$ die erste trainierte Funktion TF-1, und K,L und M sind die Ausdehnung sowohl des dreidimensionalen Realbilddatensatzes RDS-3D als auch des dreidimensionalen ersten Differenzbilddatensatzes DDS-3D bezüglich der ersten Richtung x, der zweiten Richtung y und der dritten Richtung z, gemessen jeweils in der Anzahl der Voxel. Das Argument der Funktion $F_1$ ist derart zu verstehen, dass der Intensitätswert $D_{klm}$ des dreidimensionalen ersten Differenzbilddatensatzes DDS-3D am Pixel mit den Indizes k, l und m von allen Intensitätswerten des dreidimensionalen Realbilddatensatzes RDS-3D abhängig sein kann.

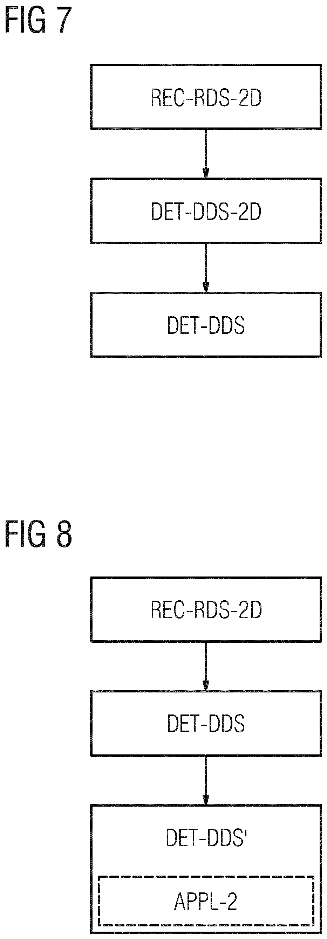

Fig. 7 zeigt ein drittes Ausführungsbeispiel des Verfahrens zum Bestimmen eines ersten Differenzbilddatensatzes DDS-3D eines Untersuchungsvolumens VOL, wobei der erste Differenzbilddatensatz dreidimensional ist. Das dritte Ausführungsbeispiel umfasst die Schritte des Empfangens REC-RDS-2D von zweidimensionalen Realbilddatensätzen RDS-2D.1, ..., RDS-2D.4 sowie das Bestimmen DET-DDS des ersten Differenzbilddatensatzes DDS-3D, diese Schritte können alle vorteilhaften Aus- und Weiterbildungen der entsprechenden Schritte des ersten oder des zweiten Ausführungsbeispiels aufweisen, soweit diese auf dieses Ausführungsbeispiel übertragbar sind. Insbesondere ist das dritte Ausführungsbeispiel auch gleichzeitig ein Ausführungsbeispiel des Verfahrens zum Bestimmen eines zweidimensionalen Differenzbilddatensatzes DDS-2D.1, ..., DDS-2D.4 eines Untersuchungsvolumens VOL, umfassend eine Vielzahl von ergänzenden optionalen Schritten, insbesondere ist hierbei der Schritt des Bestimmens DET-DDS des ersten Differenzbilddatensatzes DDS-3D optional.

Weiterhin umfasst das dritte Ausführungsbeispiel des Verfahrens zum Bestimmen eines dreidimensionalen ersten Differenzbilddatensatzes DDS-3D eines Untersuchungsvolumens VOL den Schritt des Bestimmens DET-DDS-2D von zweidimensionalen Differenzbilddatensätzen DDS-2D.1, ..., DDS-2D.4 durch Anwendung der ersten trainierten Funktion TF-2 auf die zweidimensionalen Realbilddatensätze RDS-2D.1, ..., RDS-2D.4 mittels der Recheneinheit SYS.CU. Weiterhin umfasst das Bestimmen DET-DDS des dreidimensionalen ersten Differenzbilddatensatzes DDS-3D in diesem Fall ein Rekonstruieren des dreidimensionalen ersten Differenzbilddatensatzes DDS-3D basierend auf den zweidimensionalen Differenzbilddatensätzen DDS-2D.

Im Unterschied zum ersten und zum zweiten Ausführungsbeispiel erfolgt der Übergang von Realbilddatensätzen zu Differenzbilddatensätzen im dritten Ausführungsbeispiel nicht im Dreidimensionalen (also vom dreidimensionalen Realbilddatensatz RDS-3D zum dreidimensionalen Differenzbilddatensatz DDS-3D), sondern im Zweidimensionalen (also von den zweidimensionalen Realbilddatensätzen RDS-2D.1, ..., RDS-2D.4 zu den zweidimensionalen Differenzbilddatensätzen DDS-2D.1, ..., DDS-2D.4).

In diesem Fall kann die trainierte Funktion TF-2 insbesondere basierend auf zweidimensionalen Trainingsrealbilddatensätzen und zugehörigen zweidimensionalen Trainingssubtraktionsbilddatensätzen trainiert werden, wobei die zweidimensionalen Trainingssubtraktionsbilddatensätze durch Subtraktion von jeweils einem zweidimensionalen Trainingsrealbilddatensatz und einer zugehörigen zweidimensionalen Trainingsmaskenaufnahme bestimmt werden. Hierbei werden die zweidimensionalen Trainingsrealbilddatensätze als Eingabedaten für die trainierte Funktion TF-2 verwendet, und die Ausgabedaten für die trainierte Funktion TF-2 mit dem zugehörigen zweidimensionalen Trainingssubtraktionsbilddatensätzen verglichen. Insbesondere kann für das Training dann eine geeignete vergleichende Kostenfunktion verwendet werden, beispielsweise die Summe der quadratischen Abweichungen der Pixelwerte.

Fig. 8 zeigt ein viertes Ausführungsbeispiel eines Verfahrens zum Bestimmen eines ersten Differenzbilddatensatzes DDS-3D eines Untersuchungsvolumens VOL, wobei in diesem vierten Ausführungsbeispiels auch ein vierdimensionaler zweiter Differenzbilddatensatz DDS-4D' des Untersuchungsvolumens VOL bestimmt wird.

Das vierte Ausführungsbeispiel umfasst hier die Schritte des Empfangens REC-RDS-2D von zweidimensionalen Realbilddatensätzen RDS-2D.1, ..., RDS-2D.4 sowie das Bestimmen DET-DDS des dreidimensionalen ersten Differenzbilddatensatzes DDS-3D, diese Schritte können alle vorteilhaften Aus- und Weiterbildungen der entsprechenden Schritte des ersten, des zweiten und/oder des dritten Ausführungsbeispiels aufweisen. Weiterhin kann das vierte Ausführungsbeispiel weitere Schritte und Unterschritte des ersten, des zweiten und/oder des dritten Ausführungsbeispiels umfassen.

Das vierte Ausführungsbeispiel umfasst weiterhin das Bestimmen DET-DDS' eines vierdimensionalen zweiten Differenzbilddatensatzes DDS-4D' basierend auf dem dreidimensionalen ersten Differenzbilddatensatz DDS-3D und den zweidimensionalen Realbilddatensätzen RDS-2D, oder basierend auf dem dreidimensionalen ersten Differenzbilddatensatz DDS-3D und den zweidimensionalen Differenzbilddatensätzen DDS-2D, mittels der Recheneinheit SYS.CU.

Beispielsweise kann der vierdimensionale zweite Differenzbilddatensatz DDS-4D' durch Normierung der zweidimensionalen Differenzbilddatensätze DDS-2D.1, ..., DDS-2D.4 basierend auf dem dreidimensionalen Differenzbilddatensatz DDS-3D und durch Rückprojektion (insbesondere durch multiplikative Rückprojektion) der zweidimensionalen Differenzbilddatensätzen DDS-2D.1, ..., DDS-2D.4 auf den dreidimensionalen ersten Differenzbilddatensatz DDS-3D bestimmt werden.

Die Normierung ist in diesem Ausführungsbeispiel durch den folgenden funktionalen Zusammenhang gegeben:

$$d_N(\text{t,u}) = \frac{d(\text{t,u})}{\int_{L(\text{t,u})} D(\text{l})\,d\text{l}}$$

Hierbei ist u eine zweidimensionale räumliche Koordinate im Koordinatensystem des Röntgendetektors 302, und t ist eine zeitliche Koordinate, insbesondere also eine Zeitinformation. Weiterhin bezeichnet D den dreidimensionalen ersten Differenzbilddatensatz DDS-3D, und D(x) den Wert des dreidimensionalen ersten Differenzbilddatensatzes DDS-3D an der räumlichen Koordinate x. Der eindimensionale Pfad L(t,u) entspricht der Gerade durch die punktförmige Röntgenquelle XRAY.SRC und den Punkt u auf dem Röntgendetektor XRAY.DTC zum Aufnahmezeitpunkt t. Der Pfad L(t,u) ist weiterhin von der zeitlichen Koordinate t abhängig, weil sich im Regelfall die räumliche Position der Röntgenquelle XRAY.SRC und des Röntgendetektors XRAY.DTC mit der zeitlichen Koordinate t verändern. Die Größe d(t,u) beschreibt den Intensitätswert des zum Aufnahmezeitpunkt t aufgenommenen zweidimensionalen Differenzbilddatensatzes DDS-2D.1, ..., DDS-2D.4 in der Detektorkoordinate u. Das Ergebnis $D_N(t,u)$ ist der normierte Intensitätswert des zum Aufnahmezeitpunkt t aufgenommenen zweidimensionalen Differenzbilddatensatzes DDS-2D.1, ..., DDS-2D.4 in der Detektorkoordinate u.

Weiterhin ist die multiplikative Rückprojektion durch folgenden funktionalen Zusammenhang gegeben:

$$D_{4D}(t, x) = \text{Seg}[D](x) \frac{p_N(t,\ A(t,x))}{K * \int_{L(t,\ A(t,x))} D(\text{l})\,d\text{l}}$$

Hierbei ist x eine dreidimensionale räumliche Koordinate und t eine zeitliche Koordinate, insbesondere also eine Zeitinformation. Das Tupel (t,x) kann daher auch als vierdimensionale Koordinate aufgefasst werden. Weiterhin bezeichnet D den dreidimensionalen ersten Differenzbilddatensatz DDS-3D, und D(x) den Wert des dreidimensionalen ersten Differenzbilddatensatzes DDS-3D an der räumlichen Koordinate x. Weiterhin bezeichnet A(t,x) die Projektion der räumlichen Koordinate x zum Aufnahmezeitpunkt t auf eine räumlich zweidimensionale Detektorkoordinate u = A(t,x) eines Röntgendetektors XRAY.DTC. Weiterhin bezeichnet K einen optionalen Faltungskern, der Operator * eine Faltung und Seg[D] (x) bezeichnet eine Segmentierung (bzw. den Wert dieser Segmentierung an der räumlichen Koordinate x) des dreidimensionalen ersten Differenzbilddatensatzes DDS-3D. Alternativ zur Segmentierung kann auch direkt der dreidimensionale erste Differenzbilddatensatze DDS-3D verwendet werden. Weiterhin bezeichnet $D_{4d}(t,x)$ den Wert des vierdimensionalen zweiten Differenzbilddatensatzes DDS-4D' an der räumlichen Koordinate x und an der zeitlichen Koordinate t.

Im dargestellten Ausführungsbeispiel ist die Segmentierung Seg[D] des dreidimensionalen ersten Differenzbilddatensatzes DDS-3D eine Schwellenwert-Segmentierung, es werden also alle Voxel des dreidimensionalen Differenzbilddatensatzes DDS-3D mit Hounsfield-Einheiten über dem Schwellenwert einer ersten Region bzw. einem ersten Teil zugeordnet, die insbesondere einem oder mehreren Gefäßen entsprechen kann, weiterhin werden alle Voxel des dreidimensionalen ersten Differenzbilddatensatzes DDS-3D mit Hounsfield-Einheiten unter dem Schwellenwert einer zweiten Region bzw. einer zweiten Region zugeordnet. Es sind aber auch andere Methoden zur Segmentierung möglich, beispielsweise Regionenwachstum (ein englischer Fachbegriff ist "region growing") oder aktive Formmodelle (ein englischer Fachbegriff ist "active shape models"). Das Ergebnis der Segmentierung bzw. der segmentierte dreidimensionale ersten Differenzbilddatensatz Seg[D] kann als Funktion Seg[D] aufgefasst werden, wobei die Funktion Seg[D] einem Voxel mit der räumlich dreidimensionalen Koordinate x einen Wert Seg[D] (x) zuordnet, falls der Voxel in der ersten Region liegt, wobei der Wert Seg[D](x) dem Wert des Voxels im zweiten DSA-Datensatz entspricht, und wobei die Funktion Seg[D] einem Voxel mit der räumlich dreidimensionalen Koordinate x einen Wert Seg[D](x) = 0 zuordnet, falls der Voxel in der zweiten Region liegt. Dementsprechend kann das Ergebnis einer Segmentierung bzw. der segmentierte dreidimensionale erste Differenzbilddatensatz Seg[D] wieder als Bilddatensatz aufgefasst werden.

Alternativ kann der vierdimensionale zweite Differenzbilddatensatz DDS-4D auch durch Normierung der zweidimensionalen Realbilddatensätze RDS-2D basierend auf dem dreidimensionalen ersten Differenzbilddatensatz DDS-3D und durch Rückprojektion (insbesondere durch multiplikative Rückprojektion) der zweidimensionalen Realbilddatensätze RDS-2D auf den dreidimensionalen ersten Differenzbilddatensatz DDS-3D bestimmt werden. Das Normierung und des Rückprojizieren kann dabei analog zum Fall der zweidimensionalen Differenzbilddatensätze RDS-2D durchgeführt werden.

Wiederum alternativ kann das Bestimmen DET-DDS' des vierdimensionalen zweiten Differenzbilddatensatzes DDS-4D' eine Anwendung APPL-2 einer zweiten trainierten Funktion TF-4 auf Eingabedaten umfasst, wobei die Eingabedaten auf den zweidimensionalen Realbilddatensätzen RDS-2D, den zweidimensionalen Differenzbilddatensätzen DDS-2D.1, ..., DDS-2D.4 und/oder auf dem dreidimensionalen ersten Differenzbilddatensatz DDS-3D basieren.

In diesem Ausführungsbeispiel ist die zweite trainierte Funktion TF-4 ein tiefes neuronales Netzwerk, welches als Eingabedaten die zweidimensionalen Realbilddatensätze RDS-2D und den dreidimensionalen ersten Differenzbilddatensatz DDS-3D erhält, und als Ausgabe den vierdimensionalen zweiten Differenzbilddatensatz DDS-4D' erzeugt. Diese zweite trainierte Funktion TF-4 kann insbesondere durch den Vergleich ihrer Ausgabewerte und vierdimensionaler Trainingsdifferenzbilddatensätze trainiert werden, wobei die vierdimensionalen Trainingsdifferenzbilddatensätze durch Anwendung einer Normierung und einer multiplikativen Rückprojektion basierend auf den zweidimensionalen Realbilddatensätze RDS-2D und dem dreidimensionalen ersten Differenzbilddatensatz DDS-3D bestimmt werden.

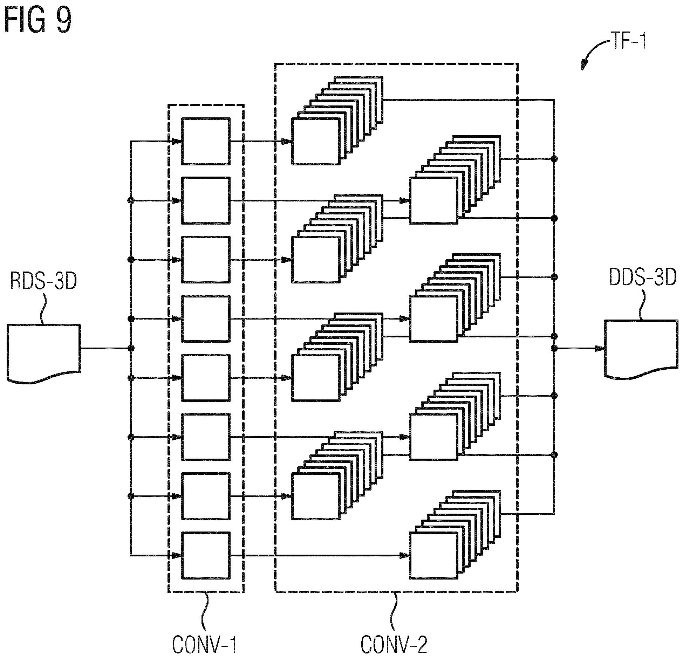

Fig. 9 zeigt ein Ausführungsbeispiel einer ersten trainierten Funktion TF-1 ...

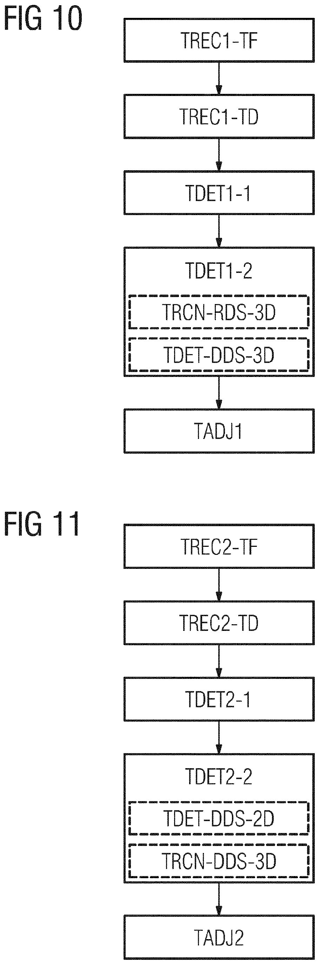

Fig. 10, Fig. 11 und Fig. 12 zeigen ein erstes Ausführungsbeispiel, ein zweites Ausführungsbeispiel und ein drittes Ausführungsbeispiel eines Verfahrens zum Trainieren einer ersten trainierten Funktion TF-1, TF-2, TF-3. Im ersten Ausführungsbeispiel bildet die erste trainierte Funktion TF-1 einen dreidimensionalen Bilddatensatz auf einen weiteren dreidimensionalen Bilddatensatz ab, insbesondere bildete die erste trainierte Funktion TF-1 in diesem Ausführungsbeispiel einen dreidimensionalen Realbilddatensatz RDS-3D auf einen dreidimensionalen ersten Differenzbilddatensatz DDS-3D ab. Im zweiten Ausführungsbeispiel bildet die erste trainierte Funktion TF-2 einen zweidimensionalen Bilddatensatz auf einen weiteren zweidimensionalen Bilddatensatz ab, insbesondere bildet die erste trainierte Funktion TF-2 in diesem Ausführungsbeispiel einen zweidimensionalen Realbilddatensatz RDS-2D.1, ..., RDS-2D.4 auf einen zweidimensionalen Differenzbilddatensatz DDS-2D.1, ..., DDS-2D.4 ab. Im dritten Ausführungsbeispiel bildet die erste trainierte Funktion TF-3 mehrere zweidimensionale Bilddatensätze auf einen vierdimensionalen Bilddatensatz ab, insbesondere bildet die erste trainierte Funktion TF-1 in diesem Ausführungsbeispiel mehrere zweidimensionale Realbilddatensätze 2D.1, ..., RDS-2D.4 auf einen vierdimensionalen ersten Differenzbilddatensatz ab.

Der erste Schritt des ersten, des zweiten und des dritten Ausführungsbeispiels ist das Empfangen TREC1-TF, TREC2-TF, TREC3-TF der ersten trainierten Funktion TF-1, TF-2, TF-3 mittels einer Schnittstelle TSYS.IF. In diesen Ausführungsbeispielen ist die erste trainierte Funktion TF-1, TF-2, TF-3 bereits vortrainiert, d.h. es wurden bereits ein oder mehrere Parameter der ersten trainierten Funktion TF-1, TF-2, TF-3 durch das beschriebene Trainingsverfahren und/oder ein anderes Trainingsverfahren angepasst. Alternativ können der eine oder die mehreren Parameter der ersten trainierten Funktion TF-1, TF-2, TF-3 noch nicht mittels Trainingsdaten angepasst sein, insbesondere können der eine oder die mehreren Parameter durch einen konstanten Wert und/oder durch einen zufälligen Wert vorbelegt sein. Insbesondere können alle Parameter der ersten trainierten Funktion TF-1, TF-2, TF-3 noch nicht mittels Trainingsdaten angepasst sein, insbesondere können alle Parameter durch einen konstanten Wert und/oder durch einen zufälligen Wert vorbelegt sein.

Der zweite Schritt des ersten, des zweiten und des dritten Ausführungsbeispiels ist das Empfangen TREC1-TD, TREC2-TD, TREC3-TD von ersten zweidimensionalen Trainingsbilddatensätzen und von zweiten zweidimensionalen Trainingsbilddatensätzen eines Untersuchungsvolumens VOL mittels der Schnittstelle TSYS.IF.

Hierbei umfasst jeder der ersten zweidimensionalen Trainingsbilddatensätze eine zweidimensionale Röntgenprojektion des Untersuchungsvolumens VOL bezüglich einer Projektionsrichtung, wobei das Untersuchungsvolumen VOL während der Aufnahme einer ersten zweidimensionalen Röntgenprojektion kein Röntgenkontrastmittel umfasst. Insbesondere kann jeder der ersten zweidimensionalen Trainingsbilddatensätze die Projektionsrichtung der jeweiligen Röntgenprojektion umfassen. Insbesondere kann jedem der ersten zweidimensionalen Trainingsbilddatensätze ein Aufnahmezeitpunkt der zugeordneten ersten Röntgenprojektion zugeordnet sein, bzw. kann jeder der ersten zweidimensionalen Trainingsbilddatensätze diesen Aufnahmezeitpunkt umfassen. Insbesondere ist also jeder der ersten zweidimensionalen Trainingsbilddatensätze ein zweidimensionaler Realbilddatensatz.

Weiterhin umfasst hierbei jeder der zweiten zweidimensionalen Trainingsbilddatensätze eine zweite zweidimensionale Röntgenprojektion des Untersuchungsvolumens VOL bezüglich einer Projektionsrichtung, wobei das Untersuchungsvolumen VOL während der Aufnahme einer zweiten Röntgenprojektion Röntgenkontrastmittel umfasst. Insbesondere kann jeder der zweiten zweidimensionalen Trainingsbilddatensätze die Projektionsrichtung der jeweiligen Röntgenprojektion umfassen. Insbesondere kann jedem der zweiten zweidimensionalen Trainingsbilddatensätze ein Aufnahmezeitpunkt der zugeordneten zweiten Röntgenprojektion zugeordnet sein, bzw. kann jeder der zweiten zweidimensionalen Trainingsbilddatensätze diesen Aufnahmezeitpunkt umfassen. Insbesondere ist also jeder der zweiten zweidimensionalen Trainingsbilddatensätze ein zweidimensionaler Realbilddatensatz.

Insbesondere kann jedem der ersten zweidimensionalen Trainingsbilddatensätze ein zweiter zweidimensionaler Trainingsbilddatensatz der zweidimensionalen Trainingsbilddatensätze zugeordnet sein, wobei die Projektionsrichtungen der jeweiligen ersten Röntgenprojektion und der jeweiligen zweiten Röntgenprojektion übereinstimmen.

Der dritte Schritt des ersten, des zweiten und des dritten Ausführungsbeispiels ist das Bestimmen TDET1-1, TDET2-1, TDET3-1 eines ersten Trainingsdifferenzbilddatensatzes $D_1(x)$ bzw. $D_1(x, t)$ durch digitale Subtraktionsangiographie basierend auf den ersten und den zweiten zweidimensionalen Trainingsbilddatensätzen mittels einer Recheneinheit TSYS.CU. Im ersten und im zweiten Ausführungsbeispiel ist der erste Trainingsdifferenzbilddatensatz $D_1(x)$ dreidimensional, im dritten Ausführungsbeispiel ist der erste Trainingsdifferenzbilddatensatz $D_1(x, t)$ vierdimensional.

In diesem Ausführungsbeispiel entsprechen die Projektionsrichtungen der Röntgenprojektionen der ersten zweidimensionalen Trainingsbilddatensätze den Projektionsrichtungen der Röntgenprojektionen der zweiten zweidimensionalen Trainingsbilddatensätze. Daher kann für jeden ersten zweidimensionalen Trainingsbilddatensatz $b_1^{(1)}(y)$, ..., $b_1^{(n)}(y)$ der ersten zweidimensionalen Trainingsbilddatensätze bzw. für jeden zweiten zweidimensionalen Trainingsbilddatensatz $b_2^{(1)}(y)$, ..., $b_2^{(n)}(y)$ der zweiten zweidimensionalen Trainingsbilddatensätze ein zweidimensionaler Trainingsdifferenzbilddatensatz $d^{(k)}(y) = b_2^{(k)}(y) - b_1^{(k)}(y)$ durch Subtraktion des ersten zweidimensionalen Trainingsbilddatensatzes $b_1^{(1)}(y)$, ..., $b_1^{(n)}(y)$ vom zweiten zweidimensionalen Trainingsbilddatensatz $b_2^{(1)}(y)$, ..., $b_2^{(n)}(y)$ berechnet werden. Basierend auf mehreren der zweidimensionalen Trainingsdifferenzbilddatensätze $d^{(1)}(y)$, ..., $d^{(n)}(y)$ kann dann durch eine digitale Subtraktionsangiographie der erste Trainingsdifferenzbilddatensatz $D_1(x)$ bzw. $D_1(x, t)$ bestimmt werden. Hierbei bezeichnet y eine zweidimensionale Koordinate, und x bezeichnet eine dreidimensionale Koordinate. Weiterhin bezeichnet im vierdimensionalen Fall t die zeitliche Koordinate. Im Folgenden wird als abkürzende Notation auch $b^{(k)}(y) := b_2^{(k)}(y)$ verwendet.

Der vierte Schritt des ersten, des zweiten und des dritten Ausführungsbeispiels ist das Bestimmen TDET1-2, TDET2-2, TDET3-2 eines zweiten Trainingsdifferenzbilddatensatzes $D_2(x)$ bzw. $D_2(x, t)$ basierend auf den zweiten zweidimensionalen Trainingsbilddatensätzen $b^{(1)}(y)$, ..., $b^{(n)}(y)$ und basierend auf der ersten trainierten Funktion TF-1, TF-2, TF-3 mittels der Recheneinheit TSYS.CU. Im ersten und im zweiten Ausführungsbeispiel ist der zweite Trainingsdifferenzbilddatensatz $D_2(x)$ dreidimensional, im dritten Ausführungsbeispiel ist der zweite Trainingsdifferenzbilddatensatz $D_2(x, t)$ vierdimensional.

Im ersten Ausführungsbeispiel wird hierbei durch Rekonstruktion TRCN-RDS-3D der zweiten zweidimensionalen Trainingsbilddatensätze $b^{(1)}(y)$, ..., $b^{(n)}(y)$ ein dreidimensionaler Trainingsbilddatensatz B(x) bestimmt, wobei der dreidimensionale Trainingsbilddatensatz B(x) ein dreidimensionaler Realbilddatensatz ist. Anschließend wird durch Anwenden TDET-DDS-3D der ersten trainierten Funktion TF-1 auf den dreidimensionalen Trainingsbilddatensatz B(x) der zweite dreidimensionale Trainingsdifferenzbilddatensatz $D_2(x)$ bestimmt, es gilt also

$$D_2(x) = TF_1(B(x)) = TF_1\left(Rec\left(b^{(1)}(y), \ldots, b^{(n)}(y)\right)\right).$$

Hierbei bezeichnet Rec den Rekonstruktionsoperator.

Im zweiten Ausführungsbeispiel wird hierbei durch Anwenden TDET-DDS-2D der ersten trainierten Funktion TF-2 aus jedem der zweiten zweidimensionalen Trainingsbilddatensätze $d^{(1)}(y)$, ..., $d^{(n)}(y)$ ein zweidimensionaler Trainingsdifferenzbilddatensatz $b^{(1)}(y)$, ..., $b^{(n)}(y)$ bestimmt, wobei jeder der zweidimensionalen Trainingsdifferenzbilddatensätze ein zweidimensionaler Differenzbilddatensatz, aber kein zweidimensionaler Subtraktionsbilddatensatz ist. Anschließend wird durch Rekonstruktion TRCN-DDS-3D der zweidimensionalen Trainingsdifferenzbilddatensätze $b^{(1)}(y)$, ..., $b^{(n)}(y)$ der zweite dreidimensionale Trainingsdifferenzbilddatensatz $D_2(x)$ bestimmt, es gilt also

$$D_2(x) = Rec\left(TF_2(d^{(1)}(y)), \ldots, TF_2(d^{(n)}(y))\right).$$

Im dritten Ausführungsbeispiel wird hierbei durch Anwenden TDET-DDS-4D der ersten trainierten Funktion TF-3 auf die Gesamtheit der zweiten zweidimensionalen Trainingsbilddatensätze $d^{(1)}(y)$, ..., $d^{(n)}(y)$ der vierdimensionale zweite Trainingsdifferenzbilddatensatz $D_2(x, t)$ bestimmt, es gilt also

$$D_2(x, t) = TF_3\left(b^{(1)}(y), \ldots, b^{(n)}(y)\right)$$

Der fünfte Schritt des ersten, des zweiten und des dritten Ausführungsbeispiels ist das Anpassen TADJ1, TADJ2, TADJ3 der ersten trainierten Funktion TF-1, TF-2, TF-3 basierend auf einem Vergleich des ersten Trainingsdifferenzbilddatensatzes $D_1(x)$ bzw. $D_1(x, t)$ und des zweiten Trainingsdifferenzbilddatensatzes $D_2(x)$ bzw. $D_2(x, t)$ mittels der Recheneinheit TSYS.CU.

Im ersten und zweiten Ausführungsbeispiel basiert der Vergleich des ersten dreidimensionalen Trainingsdifferenzbilddatensatzes $D_1(x)$ und des zweiten dreidimensionalen Trainingsdifferenzbilddatensatzes $D_2(x)$ auf der pixelweisen Differenz des ersten dreidimensionalen Trainingsdifferenzbilddatensatzes $D_1(x)$ und des zweiten dreidimensionalen Trainingsdifferenzbilddatensatzes $D_2(x)$, beispielsweise auf der Summe der quadratischen Abweichungen DIF:

$$DIF = \iiint \left(D_1(x) - D_2(x)\right)^2 dx$$

Die dreifache Integration kann hierbei auch als eine Summe über die dreidimensional angeordneten Pixel interpretiert werden. Hierbei werden dann ein oder mehrere Parameter der trainierten Funktion TF-1, TF-2 derart angepasst, dass die Summe der quadratischen Abweichungen DIF minimiert wird, beispielsweise mittels einer Rückpropagation (ein englischer Fachbegriff ist "backpropagation"). Hierbei kann es notwendig sein, die Rückpropagation nicht nur in der trainierten Funktion TF-1, TF-2 durchzuführen, sondern auch durch den jeweiligen Rekonstruktionsoperator. Hierfür sind dem Fachmann beispielsweise numerische Methoden bekannt.

Im zweiten Ausführungsbeispiel ist es alternativ auch möglich, dass ein oder mehrere Parameter basierend auf einem Vergleich der Ausgabewerte $TF_2(b_2(y))$ der zweiten trainierten Funktion TF-2 bei Anwendung auf die zweiten Trainingsbilddatensätze $b_2(y)$ und der zweidimensionalen Trainingsdifferenzbilddatensätze $d(y) = b_2(y) - b_1(y)$ angepasst wird. Insbesondere kann hierbei die Summe der quadratischen Abweichungen der Ausgabewerte $TF_2(b_2(y))$ und der zweidimensionalen Trainingsdifferenzbilddatensätze $d(y)$ durch Rückpropagation minimiert werden.

Im dritten Ausführungsbeispiel wird beim Vergleich weiterhin auch die zeitliche Dimension des ersten Trainingsdifferenzbilddatensatzes und des zweiten Trainingsdifferenzbilddatensatzes einbezogen. Beispielsweise kann die Summe der quadratischen Abweichungen berechnet werden als

$$\mathrm{DIF} = \int \left[ \iiint \left( \mathrm{D}_1(x,\ t) \text{-} \mathrm{D}_2(x,\ t) \right)^2 \mathrm{d}x \right] \mathrm{d}t.$$

Diese Summe der quadratischen Abweichungen kann ebenfalls diskret durch eine vierdimensionale Summe über alle Pixel bestimmt werden.

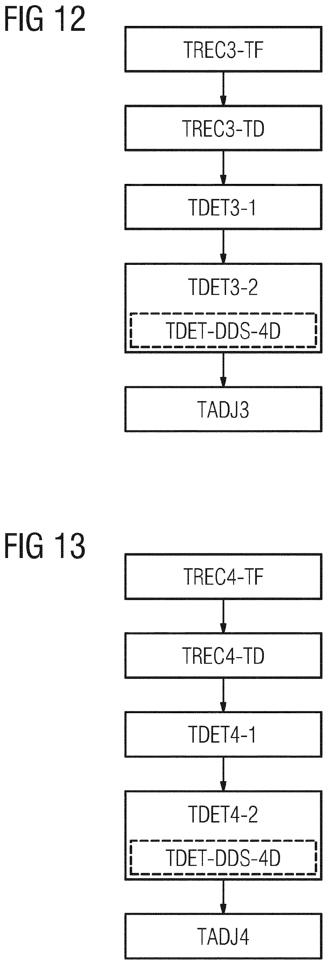

Fig. 13 zeigt ein Ausführungsbeispiel eines Verfahrens zum Anpassen einer zweiten trainierten Funktion.

Der erste Schritt des Ausführungsbeispiels ist das Empfangen TREC4-TF der zweiten trainierten Funktion TF-4 mittels einer Schnittstelle TSYS.IF. In diesem Ausführungsbeispiel bildet die zweite trainierte Funktion TF-4 einen dreidimensionalen Bilddatensatz und eine Mehrzahl von zweidimensionalen Bilddatensätze auf einen vierdimensionalen Bilddatensatz ab, insbesondere einen dreidimensionalen ersten Differenzbilddatensatz DDS-3D und eine Mehrzahl von zweidimensionalen Differenzbilddatensätzen DDS-2D.1, ..., DDS-2D.4 auf einen vierdimensionalen zweiten Differenzbilddatensatz DDS-4D'. Alternativ kann die zweite Trainierte Funktion TF-4 auch einen dreidimensionalen ersten Differenzbilddatensatz DDS-3D und eine Mehrzahl von zweidimensionalen Realbilddatensätzen RDS-2D.1, ..., RDS-2D.4 auf einen vierdimensionalen zweiten Differenzbilddatensatz DDS-4D' abbilden. In diesem Ausführungsbeispiel ist die zweite trainierte Funktion TF-2 bereits vortrainiert, d.h. es wurden bereits ein oder mehrere Parameter der zweiten trainierten Funktion TF-2 durch das beschriebene Trainingsverfahren und/oder ein anderes Trainingsverfahren angepasst. Alternativ können der eine oder die mehreren Parameter der zweiten trainierten Funktion TF-2 noch nicht mittels Trainingsdaten angepasst sein, insbesondere können der eine oder die mehreren Parameter durch einen konstanten Wert und/oder durch einen zufälligen Wert vorbelegt sein. Insbesondere können alle Parameter der zweiten trainierten Funktion TF-4 noch nicht mittels Trainingsdaten angepasst sein, insbesondere können alle Parameter durch einen konstanten Wert und/oder durch einen zufälligen Wert vorbelegt sein.

Der zweite Schritt des Ausführungsbeispiels ist das Empfangen TREC4-TD eines dreidimensionalen Trainingsdifferenzbilddatensatzes D(x) eines Untersuchungsvolumens VOL und von zweidimensionalen Trainingsbilddatensätzen des Untersuchungsvolumens VOL mittels der Schnittstelle TSYS.IF, wobei die zweidimensionalen Trainingsbilddatensätze zweidimensionalen Realbilddatensätzen $b^{(1)}(y)$, ..., $b^{(n)}(y)$ oder zweidimensionalen Differenzbilddatensätzen $d^{(1)}(y)$, ..., $d^{(n)}(y)$ entsprechen. Insbesondere umfassen die zweidimensionalen Trainingsbilddatensätze Röntgenprojektionen des Untersuchungsvolumens VOL bezüglich verschiedener Projektionsrichtungen und zugeordnete Aufnahmezeitpunkte.

Der dritte Schritt des Ausführungsbeispiels ist das Bestimmen TDET4-1 eines ersten vierdimensionalen Trainingsdifferenzbilddatensatzes $D_1(x, t)$ durch Rückprojektion basierend auf dem dreidimensionalen Trainingsdifferenzbilddatensatz D(x) und den zweidimensionalen Trainingsbilddatensätzen $b^{(1)}(y)$, ..., $b^{(n)}(y)$ bzw. $d^{(1)}(y)$, ..., $d^{(n)}(y)$ mittels einer Recheneinheit TSYS.CU. Hierbei kann die multiplikative Rückprojektion insbesondere mit dem zu Fig. 8 beschriebenen Verfahren durchgeführt werden.

Der vierte Schritt des Ausführungsbeispiels ist das Bestimmen TDET3-2 eines zweiten vierdimensionalen Trainingsdifferenzbilddatensatzes $D_2(x,t)$ durch Anwenden TDET-DDS-4D der zweiten trainierten Funktion TF-4 auf den dreidimensionalen Trainingsdifferenzbilddatensatz D(x) und die zweidimensionalen Trainingsbilddatensätze $b^{(1)}(y)$, ..., $b^{(n)}(y)$ bzw. $d^{(1)}(y)$, ..., $d^{(n)}(y)$ mittels der Recheneinheit TSYS.CU. Der zweite vierdimensionale Trainingsdifferenzbilddatensatz $D_2(x,t)$ ist dann gegeben durch

$$D_2(x,\ t) = TF_4\big(D(x), b^{(1)}(y),\ ...,\ b^{(n)}(y)\big)$$

oder durch

$$D_2(x,\ t)\ =\ TF_4\big(D(x), d^{(1)}(y),\ \ldots,\ d^{(n)}(y)\big).$$

Der fünfte Schritt des Ausführungsbeispiels ist das Anpassen TADJ4 der zweiten trainierten Funktion TF-4 basierend auf einem Vergleich des ersten vierdimensionalen Trainingsdifferenzbilddatensatzes $D_1(x,t)$ und des zweiten vierdimensionalen Trainingsdifferenzbilddatensatzes $D_2(x,t)$ mittels der Recheneinheit TSYS.CU. Das Anpassen erfolgt in diesem Ausführungsbeispiel analog zu den Schritten TADJ1, TADJ2 oder TADJ3 der Fig. 10 bis Fig. 12.

Fig. 14 zeigt eine Bestimmungssystem SYS zur Bestimmen eines dreidimensionalen Differenzbilddatensatzes DDS-3D sowie ein Trainingssystem TSYS zum Trainieren einer ersten trainierten Funktion TF-1, TF-2, TF-3 und/oder einer zweiten trainierten Funktion TF-4. Das hier dargestellte Bestimmungssystem SYS und das hier dargestellte Trainingssystem TSYS sind dazu ausgebildet, eines oder mehrere der erfindungsgemäßen Verfahren auszuführen. Das Bestimmungssystem SYS umfasst eine Schnittstelle SYS.IF, eine Recheneinheit SYS.CU sowie eine Speichereinheit SYS.MU. Das Trainingssystem TSYS umfasst eine Schnittstelle TSYS.IF, eine Recheneinheit TSYS.CU sowie eine Speichereinheit TSYS.MU.

Bei dem Bestimmungssystem SYS und/oder bei dem Trainingssystem TSYS kann es sich insbesondere um einen Computer, einen Mikrocontroller oder um einen integrierten Schaltkreis handeln. Alternativ kann es sich bei der dem Bestimmungssystem SYS und/oder bei dem Trainingssystem TSYS um einen realen oder virtuellen Verbund von Computern handeln (ein englischer Fachbegriff für einen realen Verbund ist "Cluster", ein englischer Fachbegriff für einen virtuellen Verbund ist "Cloud").

Bei einer Schnittstelle SYS.IF, TSYS.IF kann es sich um eine Hardware- oder Softwareschnittstelle handeln (beispielsweise PCI-Bus, USB oder Firewire). Eine Recheneinheit SYS.CU, TSYS.CU kann Hardware-Element oder Software-Elemente aufweisen, beispielsweise einen Mikroprozessor oder ein sogenanntes FPGA (englisches Akronym für "Field Programmable Gate Array"). Eine Speichereinheit SYS.MU, TSYS.MU kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Optimalerweise kann das Bestimmungssystem SYS und/oder das Trainingssystem TSYS weiterhin eine Ein- und Ausgabeeinheit umfassen, wobei eine Ein- und Ausgabeeinheit wenigstens eine Eingabeeinheit und/oder wenigstens eine Ausgabeeinheit umfasst.

Im gezeigten Ausführungsbeispiel ist das Bestimmungssystem SYS über ein Netzwerk NETW mit dem Trainingssystem TSYS verbunden, weiterhin ist das Bestimmungssystem SYS direkt mit einer Röntgeneinheit XRAY verbunden. Die Verbindung zur Röntgeneinheit XRAY kann aber auch mittels des Netzwerks NETW hergestellt werden. Das Bestimmungssystem SYS kann aber auch ein Teil Röntgeneinheit XRAY sein. Weiterhin kann die Kommunikation zwischen dem Bestimmungssystem SYS und dem Trainingssystem TSYS auch offline erfolgen, beispielsweise durch einen Austausch von Datenträgern. Eine Kommunikation zwischen dem Bestimmungssystem und dem Trainingssystem TSYS kann beispielsweise darin bestehen, dass das Bestimmungssystem SYS weitere Trainingsdaten an das Trainingssystem TSYS übermittelt, oder dass das Trainingssystem TSYS die erste trainierte Funktion TF-1, TF-2, TF-3 und/oder die zweite trainierte Funktion TF-4an das Bestimmungssystem SYS übermittelt. Weiterhin kann das Trainingssystem TSYS weiterhin mit anderen Datenquellen verbunden sein, insbesondere mit einem lokalen oder verteiltem PACS (englisches Akronym für "Picture Archiving and Communication System").

Das hier dargestellte Bestimmungssystem SYS ist dazu ausgebildet, die Ausführungsbeispiele des Verfahren zum Bestimmen eines dreidimensionalen Differenzbilddatensatzes DDS-3D, indem die Schnittstelle SYS.IF und die Recheneinheit SYS.CU dazu ausgebildet sind, die jeweiligen Schritte des Verfahrens auszuführen. Das hier dargestellte Trainingssystem TSYS ist dazu ausgebildet, die Ausführungsbeispiele des Verfahren zum Trainieren einer ersten trainierten Funktion TF-1, TF-2, TF-3 und/oder einer zweiten trainierten Funktion TF-4auszuführen, indem die Schnittstelle TSYS.IF und die Recheneinheit TSYS.CU dazu ausgebildet sind, die jeweiligen Schritte des Verfahrens auszuführen.

Beim Netzwerk NETW kann es sich um ein lokales Netzwerk (ein englischer Fachbegriff ist "Local Area Network", kurz "LAN") oder um ein großräumiges Netzwerk (ein englischer Fachbegriff ist "Wide Area Network", kurz "WAN") handeln. Ein Beispiel für ein lokales Netzwerk ist ein Intranet, ein Beispiel für ein großräumiges Netzwerk ist das Internet. Das Netzwerk NETW kann insbesondere auch drahtlos ausgeführt sein, insbesondere als WLAN (für "wireless LAN", im englischen ist die Abkürzung "WiFi" gebräuchlich) oder als Bluetooth-Verbindung. Das Netzwerk NETW kann auch als Kombination der genannten Beispiele ausgeführt sein.

Fig. 15 zeigt Röntgeneinheit XRAY verbunden mit einem Bestimmungssystem SYS. Im gezeigten Ausführungsbeispiel handelt es sich bei der Röntgeneinheit XRAY um ein C-Bogen-Röntgengerät XRAY. Das C-Bogen-Röntgengerät XRAY umfasst eine Röntgenquelle XRAY.SRC zum Aussenden von Röntgenstrahlen. Weiterhin umfasst das C-Bogen-Röntgengerät XRAY einen Röntgendetektor XRAY.DTC zum Empfangen von Röntgenstrahlen. Die Röntgenquelle XRAY.SRC sowie der Röntgendetektor XRAY.DTC sind an den zwei unterschiedlichen Enden des C-Bogens XRAY.ARM befestigt. Der C-Bogen XRAY.ARM des C-Bogen-Röntgengeräts XRAY ist an einem Stativ XRAY.STC befestigt. Das Stativ XRAY.STC umfasst Antriebselemente, die dazu ausgelegt sind, die Position des C-Bogens XRAY.ARM zu verändern. Insbesondere kann der C-Bogen XRAY.ARM um zwei verschiedene Achsen gedreht werden. Das C-Bogen-Röntgengerät umfasst weiterhin eine Steuer- und Auswerteeinheit XRAY.CTRL sowie eine Patientenlagerungsvorrichtung XRAY.PAT, auf der ein Patient PAT gelagert werden kann. Mittels der Steuer- und Auswerteeinheit XRAY.CTRL kann die Position des C-Bogens XRAY.ARM eingestellt werden und der C-Bogen XRAY.ARM um das Untersuchungsvolumen VOL rotiert werden. Weiterhin können mittels der Steuer- und Auswerteeinheit XRAY.CTRL zweidimensionale Röntgenprojektionen des ersten Untersuchungsvolumens VOL aufgenommen und ausgewertet werden. Es ist alternativ zum dargestellten Ausführungsbeispiel auch möglich, dass das Bestimmungssystem SYS als Teil der Steuer- und Auswerteeinheit XRAY.CTRL ausgeführt ist.

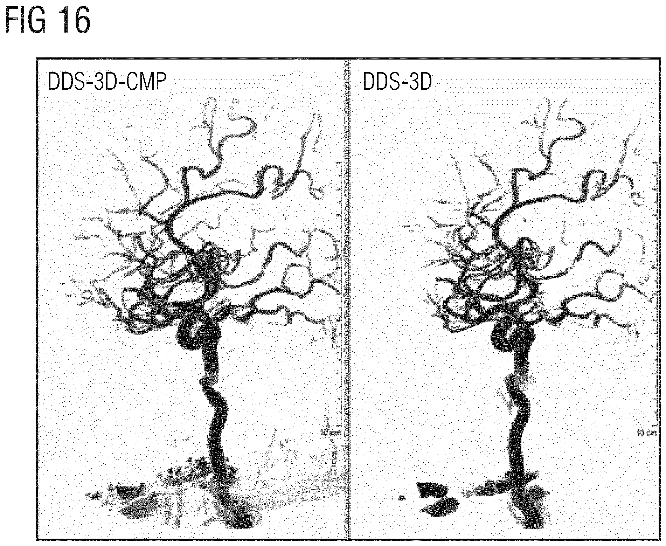

Fig. 16 zeigt eine Abbildung eines dreidimensionalen ersten Differenzbilddatensatzes DDS-3D sowie eine Abbildung eines Vergleichsbilddatensatz DDS-3D-CMP, wobei der Vergleichsbilddatensatz dreidimensional ist. Der dreidimensionale erste Differenzbilddatensatz DDS-3D wurde mit dem in Fig. 6 dargestellten und beschriebenen Ausführungsbeispiel des Verfahrens zum Bestimmen eines Differenzbilddatensatzes DDS-3D basierend auf zweidimensionalen Realbilddatensätzen RDS-2D.1, ..., RDS-2D.4 bestimmt, wobei keine Maskenaufnahmen verwendet wurden. Der Vergleichsbilddatensatz wurde basierend auf den zweidimensionalen Realbilddatensätzen RDS-2D.1, ..., RDS-2D.4 sowie Maskenaufnahmen durch bekannte digitale Subtraktionsangiographie bestimmt.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zum Bestimmen eines ersten Differenzbilddatensatzes (DDS-2D.1, ..., DDS-2D.4, DDS-3D, DDS-4D) eines Untersuchungsvolumens (VOL), umfassend:

   - Empfangen (REC-RDS-2D) von zweidimensionalen Realbilddatensätzen (RDS-2D.1, ..., RDS-2D.4) betreffend das Untersuchungsvolumen (VOL) mittels einer Schnittstelle (SYS.IF),
   wobei jeder der zweidimensionalen Realbilddatensätze (RDS-2D) eine zweidimensionale Röntgenprojektion des Untersuchungsvolumens bezüglich einer Projektionsrichtung umfasst,
   - Bestimmen (DET-DDS) des ersten Differenzbilddatensatzes (DDS-2D.1, ..., DDS-2D.4, DDS-3D, DDS-4D) basierend auf den zweidimensionalen Realbilddatensätzen (RDS-2D.1, ..., RDS-2D.4) und basierend auf einer ersten trainierten Funktion (TF-1, TF-2, TF-3) mittels einer Recheneinheit (SYS.CU) wobei der erste Differenzbilddatensatz (DDS-2D.1, ..., DDS-2D.4, DDS-3D, DDS-4D) mindestens zweidimensional, insbesondere mindestens dreidimensional ist.

2. Verfahren nach Anspruch 1, wobei der erste Differenzbilddatensatz (DDS-3D) dreidimensional ist, weiterhin umfassend:

   - Rekonstruieren (RCN-RDS-3D) eines dreidimensionalen Realbilddatensatzes (RDS-3D) basierend auf den zweidimensionalen Realbilddatensätzen (RDS-2D.1, ..., RDS-2D.4) mittels der Recheneinheit (SYS.CU),

   wobei das Bestimmen (DET-DDS) des dreidimensionalen ersten Differenzbilddatensatzes (DDS-3D) ein Anwenden (APPL-1) der ersten trainierten Funktion (TF-1) auf den dreidimensionalen Realbilddatensatz (RDS-3D) mittels der Recheneinheit (SYS.CU) umfasst.

3. Verfahren nach Anspruch 2, weiterhin umfassend:

   - Bestimmen (DET-PDS-3D) eines dreidimensionalen Wahrscheinlichkeitsdatensatzes (PDS-3D) durch Anwendung der ersten trainierten Funktion (TF-1) auf den dreidimensionalen Realbilddatensatz (RDS-3D) mittels der Recheneinheit (SYS.CU), wobei das Bestimmen (DET-DDS) des dreidimensionalen ersten Differenzbilddatensatzes (DDS-3D) ein pixelweises Multiplizieren (MPL) des dreidimensionalen Wahrscheinlichkeitsdatensatzes (PDS-3D) mit dem dreidimensionalen Realbilddatensatz (RDS-3D) mittels der Recheneinheit (SYS.CU) umfasst.

4. Verfahren nach Anspruch 3, weiterhin umfassend:

- Empfangen (REC-TF) einer Transferfunktion mittels der Schnittstelle (SYS.IF),
- Modifizieren (MOD-PDS-3D) des dreidimensionalen Wahrscheinlichkeitsdatensatzes (PDS-3D) basierend auf der Transferfunktion mittels der Recheneinheit (SYS.CU).

5. Verfahren nach Anspruch 1, wobei der erste Differenzbilddatensatz (DDS-3D) dreidimensional ist, weiterhin umfassend:

- Bestimmen (DET-DDS-2D) von zweidimensionalen Differenzbilddatensätzen (DDS-2D.1, ..., DDS-2D.4) durch Anwendung der ersten trainierten Funktion (TF-2) auf die zweidimensionalen Realbilddatensätze (RDS-2D.1, ..., RDS-2D.4) mittels der Recheneinheit (SYS.CU),

wobei das Bestimmen (DET-DDS) des dreidimensionalen ersten Differenzbilddatensatzes (DDS-3D) ein Rekonstruieren basierend auf den zweidimensionalen Differenzbilddatensätzen (DDS-2D.1, ..., DDS-2D.4) umfasst.

6. Verfahren nach Anspruch 1, wobei der erste Differenzbilddatensatz (DDS-4D) vierdimensional ist, wobei das Bestimmen des vierdimensionalen ersten Differenzbilddatensatzes (DDS-4D) durch Anwenden der ersten trainierten Funktion (TF-3) auf die zweidimensionalen Realbilddatensätze (RDS-2D.1, ..., RDS-2D.4) erfolgt.

7. Verfahren nach einem der Ansprüche 2 bis 5, weiterhin umfassend:

- Bestimmen (DET-DDS') eines zweiten Differenzbilddatensatzes (DDS-4D') durch Anwendung einer zweiten trainierten Funktion (TF-4) auf Eingabedaten, wobei die Eingabedaten auf den zweidimensionalen Realbilddatensätzen (RDS-2D.1, ..., RDS-2D.4), den zweidimensionalen Differenzbilddatensätzen (DDS-2D.1, ..., DDS-2D.4) und/oder auf dem dreidimensionalen ersten Differenzbilddatensatz (DDS-3D) basieren,

wobei der zweite Differenzbilddatensatz (DDS-4D') vierdimensional ist.

8. Verfahren nach einem der vorstehenden Ansprüche, wobei die erste trainierte Funktion (TF-1, TF-2, TF-3) und/oder die zweite trainierte Funktion (TF-4) auf einem neuronalen Netzwerk basieren.

9. Verfahren nach Anspruch 8, wobei das neuronale Netzwerk eine Faltungsschicht und/oder eine Entfaltungsschicht umfasst.

10. Verfahren zum Bestimmen eines zweidimensionalen Differenzbilddatensatzes (DDS-2D.1, ..., DDS-2D.4) eines Untersuchungsvolumens (VOL), umfassend:

- Empfangen (REC-RDS-2D) eines zweidimensionalen Realbilddatensatzes (RDS-2D.1, ..., RDS-2D.4) betreffend das Untersuchungsvolumen (VOL) mittels einer Schnittstelle (SYS.IF), wobei der zweidimensionale Realbilddatensatz (RDS-2D.1, ..., RDS-2D.4) eine zweidimensionale Röntgenprojektion des Untersuchungsvolumens bezüglich einer Projektionsrichtung umfasst,
- Bestimmen (DET-DDS-2D) des zweidimensionalen Differenzbilddatensatzes (DDS-2D.1, ..., DDS-2D.4) durch Anwendung einer ersten trainierten Funktion (TF-2) auf den zweidimensionalen Realbilddatensatz (RDS-2D.1, ..., RDS-2D.4) mittels einer Recheneinheit (SYS.CU).

11. Verfahren zum Anpassen (TADJ1, TADJ2) einer ersten trainierten Funktion (TF-1, TF-2, TF-3), umfassend:

- Empfangen (TREC1-TF, TREC2-TF) der ersten trainierten Funktion (TF-1, TF-2) mittels einer Schnittstelle (TSYS.IF),
- Empfangen (TREC1-TD, TREC2-TD) von ersten zweidimensionalen Trainingsbilddatensätzen und von zweiten zweidimensionalen Trainingsbilddatensätzen eines Untersuchungsvolumens (VOL) mittels der Schnittstelle (TSYS.IF),

wobei jeder der ersten zweidimensionalen Trainingsbilddatensätze eine zweidimensionale Röntgenprojektion des Untersuchungsvolumens (VOL) bezüglich einer Projektionsrichtung umfasst, wobei das Untersuchungsvolumen (VOL) während der Aufnahme einer ersten zweidimensionalen Röntgenprojektion kein Röntgenkontrastmittel umfasst, wobei jeder der zweiten zweidimensionalen Trainingsbilddatensätze eine zweite zweidimensionale Röntgenprojektion des Untersuchungsvolumens (VOL) bezüglich einer Projektionsrichtung umfasst,

wobei das Untersuchungsvolumen (VOL) während der Aufnahme einer zweiten Röntgenprojektion (TDS-2D-2) Röntgenkontrastmittel umfasst,

- Bestimmen (TDET1-1, TDET2-1) eines ersten Trainingsdifferenzbilddatensatzes durch digitale Subtraktionsangiographie basierend auf den ersten und den zweiten zweidimensionalen Trainingsbilddatensätzen mittels einer Recheneinheit (TSYS.CU),
- Bestimmen (TDET1-2, TDET2-2) eines zweiten Trainingsdifferenzbilddatensatzes basierend auf den zweiten zweidimensionalen Trainingsbilddatensätzen und basierend auf der ersten trainierten Funktion (TF-1, TF-2, TF-3) mittels der Recheneinheit (TSYS.CU),
- Anpassen (TADJ1, TADJ2) der ersten trainierten Funktion (TF-1, TF-2, TF-3) basierend auf einem Vergleich des ersten Trainingsdifferenzbilddatensatzes und des zweiten Trainingsdifferenzbilddatensatzes mittels der Recheneinheit (TSYS.CU).

12. Bestimmungssystem (SYS) zum Bestimmen eines ersten Differenzbilddatensatzes (DDS-2D.1, ..., DDS-2D.4, DDS-3D, DDS-4D) eines Untersuchungsvolumens (VOL), umfassend:

- Schnittstelle (SYS.IF), ausgebildet zum Empfangen (REC-RDS-2D) von zweidimensionalen Realbilddatensätzen (RDS-2D.1, ..., RDS-2D.4) betreffend das Untersuchungsvolumen (VOL), wobei jeder der zweidimensionalen Realbilddatensätze (RDS-2D.1, ..., RDS-2D.4) eine zweidimensionale Röntgenprojektion des Untersuchungsvolumens bezüglich einer Projektionsrichtung umfasst,
- Recheneinheit (SYS.CU), ausgebildet zum Bestimmen (DET-DDS) des ersten Differenzbilddatensatzes (DDS-2D.1, ..., DDS-2D.4, DDS-3D, DDS-4D) basierend auf den zweidimensionalen Realbilddatensätzen (RDS-2D.1, ..., RDS-2D.4) und basierend auf einer ersten trainierten Funktion (TF-1, TF-2, TF-3), wobei der erste Differenzbilddatensatz (DDS-2D.1, ..., DDS-2D.4, DDS-3D, DDS-4D) mindestens zweidimensional, insbesondere mindestens dreidimensional ist.

13. Röntgeneinheit (XRAY) umfassend ein Bestimmungssystem (SYS) nach Anspruch 12.

14. Trainingssystem zum Anpassen (TADJ1, TADJ2) einer erste trainierten Funktion (TF-1, TF-2, TF-3), umfassend:

- Schnittstelle (TSYS.IF), ausgebildet zum Empfangen (TREC1-TF, TREC2-TF) der trainierten Funktion (TF-1, TF-2), weiterhin ausgebildet zum Empfangen (TREC1-TD, TREC2-TD) von ersten zweidimensionalen Trainingsbilddatensätzen und von zweiten zweidimensionalen Trainingsbilddatensätzen eines Untersuchungsvolumens (VOL), wobei jeder der ersten zweidimensionalen Trainingsbilddatensätze eine zweidimensionale Röntgenprojektion des Untersuchungsvolumens (VOL) bezüglich einer Projektionsrichtung umfasst, wobei das Untersuchungsvolumen (VOL) während der Aufnahme einer ersten zweidimensionalen Röntgenprojektion kein Röntgenkontrastmittel umfasst, wobei jeder der zweiten zweidimensionalen Trainingsbilddatensätze eine zweite zweidimensionale Röntgenprojektion des Untersuchungsvolumens (VOL) bezüglich einer Projektionsrichtung umfasst, und wobei das Untersuchungsvolumen (VOL) während der Aufnahme einer zweiten Röntgenprojektion (TDS-2D-2) Röntgenkontrastmittel umfasst,
- Recheneinheit (TSYS.CU), ausgebildet zum Bestimmen (TDET1-1, TDET2-1) eines ersten Trainingsdifferenzbilddatensatzes durch digitale Subtraktionsangiographie basierend auf den ersten und den zweiten zweidimensionalen Trainingsbilddatensätzen,

weiterhin ausgebildet zum Bestimmen (TDET1-2, TDET2-2) eines zweiten Trainingsdifferenzbilddatensatzes basierend auf den zweiten zweidimensionalen Trainingsbilddatensätzen und basierend auf der ersten trainierten Funktion (TF-1, TF-2, TF-3), weiterhin ausgebildet zum Anpassen (TADJ1, TADJ2) der ersten trainierten Funktion (TF-1, TF-2, TF-3) basierend auf einem Vergleich des ersten Trainingsdifferenzbilddatensatzes und des zweiten Trainingsdifferenzbilddatensatzes.

15. Computerprogrammprodukt mit einem Computerprogramm, welches direkt in einen Speicher (SYS.MU, TSYS.MU) eines Bestimmungssystems (SYS) und/oder eines Trainingssystems (TSYS) ladbar ist, mit Programmabschnitten, um alle Schritte des Verfahrens zum Bestimmen eines Differenzbilddatensatzes (DDS-3D, DDS-4D) nach einem der Ansprüche 1 bis 9 und/oder um alle Schritte des Verfahrens zum Bestimmen eines zweidimensionalen Differenzbilddatensatzes (DDS-2D.1, ..., DDS-2D.4) und/oder um alle Schritte des Verfahrens zum Trainieren einer ersten trainierten Funktion (TF-1, TF-2, TF-3) nach dem Anspruch 11 auszuführen, wenn die Programmabschnitte von dem Bestimmungssystem (SYS) und/oder dem Trainingssystem (TSYS) ausgeführt werden.

16. Computerlesbares Speichermedium, auf welchem von einem Bestimmungssystem (SYS) und/oder einem Trainingssystem (TSYS) lesbare und ausführbare Programmabschnitte gespeichert sind, um alle Schritte des Verfahrens zum Bestimmen eines Differenzbilddatensatzes (DDS-3D, DDS-4D) nach einem der Ansprüche 1 bis 9 und/oder um alle Schritte des Verfahrens zum Bestimmen eines zweidimensionalen Differenzbilddatensatzes (DDS-2D.1, ..., DDS-2D.4) und/oder um alle Schritte des Verfahrens zum Trainieren einer ersten trainierten Funktion (TF-1, TF-2, TF-3) nach dem Anspruch 11 auszuführen, wenn die Programmabschnitte von dem Bestimmungssystem (SYS) und/oder dem Trainingssystem (TSYS) ausgeführt werden.

FIG 1

FIG 2

VES.1　　VES.2　　VES.1

RDS-2D.1

OS.1

OS.1

OS.2

OS.2

CA.1

CA.1

RDS-2D.2

CA.2

RDS-2D.3
VES.2

VES.1

VES.1
VES.2

RDS-2D.4

OS.2

OS.1

OS.2

OS.1

CA.4

OS.1

CA.3　　CA.3

FIG 3

VES.1　　VES.2　　VES.1

DDS-2D.1

DDS-2D.2

CA.2

CA.1

CA.1

DDS-2D.3
VES.2

VES.1

VES.1
VES.2

DDS-2D.4

CA.4

CA.3　　CA.3

## FIG 4

RDS-2D.1...RDS-2D.4

TF-2

RDS-3D

DDS-2D.1...DDS-2D.4

TF-1

TF-4

DDS-3D

TF-3

DDS-4D, DDS-4D'

## FIG 5

REC-RDS-2D

DET-DDS

# FIG 6

```
                    ┌─────────────────────┐
                    │    REC-RDS-2D        │
                    └──────────┬──────────┘
                               │
                               ▼
                    ┌─────────────────────┐
                    │    RCN-RDS-3D        │
                    └──────────┬──────────┘
                               │
                               ▼
        ┌──────────────────────────────────────────────────────┐
        │                   DET-DDS                             │
        │   ┌───────────────────────────────┐                  │
        │   │      DET-PDS-3D               │                   │
        │   │   ┌───────────────────────┐   │                  │
        │   │   │      APPL-1            │   │                  │
        │   │   └───────────┬───────────┘   │                  │
        │   └───────────────┼───────────────┘                  │
        │                   │      ┌─────────────────────┐     │
        │                   │      │                     │     │
        │                   │      ▼                     │     │
        │                   │   ┌──────────────────────┐ │     │
        │                   │   │      REC-TF           │ │     │
        │                   │   └──────────┬───────────┘ │     │
        │                   │              │             │     │
        │                   │              ▼             │     │
        │                   │   ┌──────────────────────┐ │     │
        │                   │   │     MOD-PDS-3D        │ │     │
        │                   │   └──────────┬───────────┘ │     │
        │                   │              │             │     │
        │                   ▼              │             │     │
        │   ┌───────────────────────────┐ │                   │
        │   │          MPL              │ │                    │
        │   └───────────────────────────┘                     │
        └──────────────────────────────────────────────────────┘
```

FIG 7

```
┌─────────────────────┐
│     REC-RDS-2D      │
└──────────┬──────────┘
           │
           ▼
┌─────────────────────┐
│     DET-DDS-2D      │
└──────────┬──────────┘
           │
           ▼
┌─────────────────────┐
│      DET-DDS        │
└─────────────────────┘
```

FIG 8

```
┌─────────────────────┐
│     REC-RDS-2D      │
└──────────┬──────────┘
           │
           ▼
┌─────────────────────┐
│      DET-DDS        │
└──────────┬──────────┘
           │
           ▼
┌─────────────────────┐
│      DET-DDS'       │
│  ┌───────────────┐  │
│  │     APPL-2    │  │
│  └───────────────┘  │
└─────────────────────┘
```

FIG 9

RDS-3D

CONV-1

CONV-2

TF-1

DDS-3D

FIG 10

```
┌─────────────────────────┐
│        TREC1-TF         │
└─────────────────────────┘
             │
             ▼
┌─────────────────────────┐
│        TREC1-TD         │
└─────────────────────────┘
             │
             ▼
┌─────────────────────────┐
│        TDET1-1          │
└─────────────────────────┘
             │
             ▼
┌─────────────────────────┐
│        TDET1-2          │
│  ┌───────────────────┐  │
│  │   TRCN-RDS-3D     │  │
│  └───────────────────┘  │
│  ┌───────────────────┐  │
│  │   TDET-DDS-3D     │  │
│  └───────────────────┘  │
└─────────────────────────┘
             │
             ▼
┌─────────────────────────┐
│         TADJ1           │
└─────────────────────────┘
```

FIG 11

```
┌─────────────────────────┐
│        TREC2-TF         │
└─────────────────────────┘
             │
             ▼
┌─────────────────────────┐
│        TREC2-TD         │
└─────────────────────────┘
             │
             ▼
┌─────────────────────────┐
│        TDET2-1          │
└─────────────────────────┘
             │
             ▼
┌─────────────────────────┐
│        TDET2-2          │
│  ┌───────────────────┐  │
│  │   TDET-DDS-2D     │  │
│  └───────────────────┘  │
│  ┌───────────────────┐  │
│  │   TRCN-DDS-3D     │  │
│  └───────────────────┘  │
└─────────────────────────┘
             │
             ▼
┌─────────────────────────┐
│         TADJ2           │
└─────────────────────────┘
```

FIG 12

```
┌─────────────────────────┐
│       TREC3-TF          │
└─────────────────────────┘
             │
             ▼
┌─────────────────────────┐
│       TREC3-TD          │
└─────────────────────────┘
             │
             ▼
┌─────────────────────────┐
│       TDET3-1           │
└─────────────────────────┘
             │
             ▼
┌─────────────────────────┐
│       TDET3-2           │
│  ┌ ─ ─ ─ ─ ─ ─ ─ ─ ─ ┐  │
│     TDET-DDS-4D         │
│  └ ─ ─ ─ ─ ─ ─ ─ ─ ─ ┘  │
└─────────────────────────┘
             │
             ▼
┌─────────────────────────┐
│       TADJ3             │
└─────────────────────────┘
```

FIG 13

```
┌─────────────────────────┐
│       TREC4-TF          │
└─────────────────────────┘
             │
             ▼
┌─────────────────────────┐
│       TREC4-TD          │
└─────────────────────────┘
             │
             ▼
┌─────────────────────────┐
│       TDET4-1           │
└─────────────────────────┘
             │
             ▼
┌─────────────────────────┐
│       TDET4-2           │
│  ┌ ─ ─ ─ ─ ─ ─ ─ ─ ─ ┐  │
│     TDET-DDS-4D         │
│  └ ─ ─ ─ ─ ─ ─ ─ ─ ─ ┘  │
└─────────────────────────┘
             │
             ▼
┌─────────────────────────┐
│       TADJ4             │
└─────────────────────────┘
```

## FIG 14

```
┌─────────────────────┐                              ┌─────────────────────┐
│         SYS         │                              │        TSYS         │
│  ┌───────────────┐  │                              │  ┌───────────────┐  │
│  │    SYS.IF     │  │                              │  │    TSYS.IF    │  │
│  └───────────────┘  │        ╭─────────╮           │  └───────────────┘  │
│  ┌───────────────┐  │        │  NETW   │           │  ┌───────────────┐  │
│  │    SYS.CU     │──┼────────┤         ├───────────┼──│   TSYS.CU     │  │
│  └───────────────┘  │        ╰─────────╯           │  └───────────────┘  │
│  ┌───────────────┐  │                              │  ┌───────────────┐  │
│  │    SYS.MU     │  │                              │  │   TSYS.MU     │  │
│  └───────────────┘  │                              │  └───────────────┘  │
└──────────┬──────────┘                              └─────────────────────┘
           │
   ┌───────┴───────┐
   │     XRAY      │
   └───────────────┘
```

## FIG 15

XRAY.ARM  
XRAY.DTC  
XRAY.STC  
PAT  VOL  
XRAY.PAT  
XRAY.SRC  
XRAY.CTRL  
SYS  
SYS.IF  
SYS.CU  
SYS.MU

FIG 16

**Europäisches Patentamt**
European Patent Office
Office européen des brevets

# EUROPÄISCHER RECHERCHENBERICHT

**Nummer der Anmeldung**

EP 18 18 2251

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2018/120644 A1 (SHANGHAI UNITED IMAGING HEALTHCARE CO LTD [CN]) 5. Juli 2018 (2018-07-05) | 1-3, 5-10,12, 13,15,16 | INV. G06T11/00 A61B6/03 A61B6/00 |
| A | * Absatz [0004] - Absatz [0006] * * Absatz [0084] - Absatz [0147] * * Absatz [0166] - Absatz [0167] * ----- | 4 | |
| X | MONTOYA JUAN C ET AL: "Deep learning angiography (DLA): three-dimensional C-arm cone beam CT angiography generated from deep learning method using a convolutional neural network", PROGRESS IN BIOMEDICAL OPTICS AND IMAGING, SPIE - INTERNATIONAL SOCIETY FOR OPTICAL ENGINEERING, BELLINGHAM, WA, US, Bd. 10573, 9. März 2018 (2018-03-09), Seiten 105731N1-105731N9, XP060104906, ISSN: 1605-7422, DOI: 10.1117/12.2293985 ISBN: 978-1-5106-0027-0 * Seite 105731N1 - Seite 105731N5 * ----- | 11,14-16 | |
| A | EP 3 340 178 A1 (SIEMENS HEALTHCARE GMBH [DE]) 27. Juni 2018 (2018-06-27) * Zusammenfassung * ----- | 1-16 | **RECHERCHIERTE SACHGEBIETE (IPC)** G06T A61B |
| A | DE 10 2014 213408 A1 (SIEMENS AG [DE]) 14. Januar 2016 (2016-01-14) * Absatz [0001] - Absatz [0072] * ----- | 1-16 | |

Der vorliegende Recherchenbericht wurde für alle Patentansprüche erstellt

| Recherchenort | Abschlußdatum der Recherche | Prüfer |
|---|---|---|
| München | 16. November 2018 | Maier, Werner |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 18 2251

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-11-2018

| Im Recherchenbericht angeführtes Patentdokument | Datum der Veröffentlichung | Mitglied(er) der Patentfamilie | Datum der Veröffentlichung |
|---|---|---|---|
| WO 2018120644 A1 | 05-07-2018 | CN 106682636 A<br>WO 2018120644 A1 | 17-05-2017<br>05-07-2018 |
| EP 3340178 A1 | 27-06-2018 | CN 108242067 A<br>DE 102016226195 B3<br>EP 3340178 A1<br>US 2018182132 A1 | 03-07-2018<br>01-02-2018<br>27-06-2018<br>28-06-2018 |
| DE 102014213408 A1 | 14-01-2016 | DE 102014213408 A1<br>US 2016012636 A1 | 14-01-2016<br>14-01-2016 |

EPO FORM P0461

Für nähere Einzelheiten zu diesem Anhang : siehe Amtsblatt des Europäischen Patentamts, Nr.12/82